(19) Europäisches Patentamt
European Patent Office
Office européen des brevets

(11) Veröffentlichungsnummer: **0 584 487 A2**

(12) **EUROPÄISCHE PATENTANMELDUNG**

(21) Anmeldenummer: **93110182.8**

(22) Anmeldetag: **25.06.93**

(51) Int. Cl.5: **C07D 487/04**, C07D 471/14, A61K 31/495, //(C07D487/04, 241:00,209:00),(C07D471/14, 241:00,221:00,209:00)

(30) Priorität: **24.08.92 DE 4228095**

(43) Veröffentlichungstag der Anmeldung: **02.03.94 Patentblatt 94/09**

(84) Benannte Vertragsstaaten: **AT BE CH DE DK ES FR GB GR IE IT LI LU MC NL PT SE**

(71) Anmelder: **ASTA Medica Aktiengesellschaft**
**An der Pikardie 10**
**D-01277 Dresden(DE)**

(72) Erfinder: **Dieter, Hans-Reinhold, Dr.**
**Gehmerweg 1**
**D-6100 Darmstadt(DE)**
Erfinder: **Engel, Jürgen, Dr.**
**Erlenweg 3**
**D-8755 Alzenau(DE)**
Erfinder: **Klingler, Karl-Heinz, Dr.**
**Beethovenstrasse 27**
**D-6070 Langen(DE)**
Erfinder: **Kutscher, Bernhard, Dr.**
**Stresemannstrasse 9**
**D-6457 Maintnal 1(DE)**
Erfinder: **Szelenyi, Stefan, Prof.**
**Händelstrasse 32**
**D-8501 Schwaig(DE)**
Erfinder: **Achterrath-Tuckermann, Ute, Dr.**
**Bahnhofstrasse 120 a**
**D-6457 Maintal 1(DE)**
Erfinder: **Schmidt, Jürgen, Dr.**
**Herzbergstrasse 38**
**D-6466 Gründau 1(DE)**
Erfinder: **Metzenauer, Peter, Dr.**
**Taunusstrasse 9**
**D-6466 Gründau 3(DE)**

(54) **Neue 4,5-Dihydro-4-oxo-pyrrolo (1,2-a) chinoxaline und entsprechende Aza-analoga und Verfahren zu deren Herstellung.**

(57) Die Erfindung betrifft neue Verbindungen der Formel

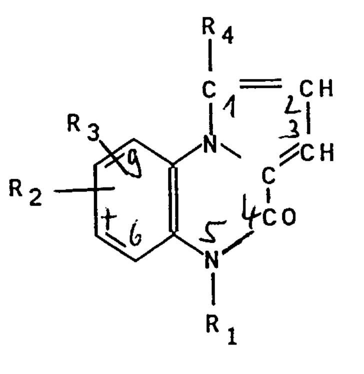

I

worin der Phenylring anstelle einer CH-Gruppe auch ein Stickstoffatom enthalten kann, $R_1$ $C_2$-$C_6$-Alkenyl, $C_2$-$C_6$-Alkinyl, Hydroxy, $C_1$-$C_6$-Alkoxy, $C_3$-$C_6$-Alkenyloxy, $C_3$-$C_6$-Alkinyloxy, $C_2$-$C_6$-Alkanoyloxy, Benzoyloxy, Morpholinocarbonyloxy,

EP 0 584 487 A2

$C_1$-$C_6$-Alkyloxycarbonyloxy,
$C_1$-$C_6$-Alkylaminocarbonyloxy,
$C_1$-$C_6$-Dialkylaminocarbonyloxy oder die Gruppe -Alk-A ist, worin Alk: $C_1$-$C_6$-Alkyl, $C_2$-$C_6$-Hydroxy-alkyl oder $C_3$-$C_6$-Cycloalkyl bedeutet und das Symbol A Wasserstoff oder andere Substituenten darstellt.

$R_2$, $R_3$ und $R_4$ verschiedene Substituenten darstellen, wobei $R_1$ auch Wasserstoff sein kann, wenn $R_2$ die Gruppe

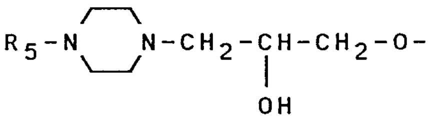

ist und $R_5$ Phenyl, $C_1$-$C_4$-Alkoxy-phenyl oder Diphenylmethyl darstellen und $R_3$ und $R_4$ Wasserstoff sind und deren Salze.

Weiterhin betrifft die Erfindung ein neues Verfahren zur Herstellung von Ausgangsstoffen für die Verbindungen der Formel I.

Die Europa-Patentanmeldung 400 583 betrifft Imidochioxaline und deren Aza-Analoge der folgenden allgemeinen Formel

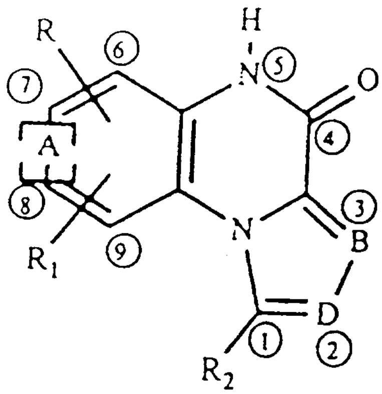

worin A ein Stickstoffatom oder CH, B und D ein Stickstoffatom oder CH, B und D ein Stickstoffatom oder CH beziehungsweise ein substituiertes Kohlenstoffatom bedeuten und die Reste R, $R_1$ und $R_2$ Wasserstoff oder verschiedene organische Substituenten darstellen.

Für diese Verbindungen wird eine positiv inotrope gefaßerweiternde Wirkung angegeben.

Weiterhin wird in Indian Journal of Chemistry, Band 10, 1972, Seiten 344-350 unter anderem die Herstellung von Verbindungen der Formel

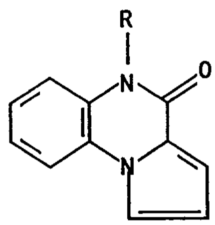

$$R = -(CH_2)_nN$$

beschrieben, worin der Rest R 3-Dimethylaminopropyl-(1), 2-Morpholino-ethyl-(1), 2-Pyrrolidinoethyl-(1) oder 2-Dimethylamino-ethyl-(1) sein kann. Eine pharmakologische Wirkung wird nicht angegeben.

Die Erfindung betrifft die durch die Patentansprüche angegebenen Gegenstände.

Die erfindungsgemäßen neuen Verbindungen sind pharmakologisch wirksam insbesondere besitzen sie antiallergische und antiasthmatische Wirkungen, anxiolytische Wirkungen, hypotensive Wirkungen (zum Beispiel selektive $\alpha_2$-Blokade), vasodilatierende Wirkungen (inodilatorisch) sowie eine positiv inotrope Wirkung (selektive PDE-III-Hemmung).

Der Erfindung liegt die Aufgabe zugrunde, neue Verbindungen mit günstigen und verbesserten pharmakologischen Eigenschaften zur Verfügung zu stellen.

Die Erfindung betrifft darüberhinaus ein neues verbessertes Verfahren zur Herstellung von Ausgangsstoffen der Formel I, worin $R_1$ und $R_4$ Wasserstoff sind und die Reste $R_2$ und $R_3$ gleich oder verschieden sind und Wasserstoff, Halogen, $C_1$-$C_6$-Alkyl, $-CF_3$, -CN, $C_1$-$C_6$-Alkoxy, Amino, $C_2$-$C_6$-Alkanoylamino, $-NO_2$, Hydroxy, $-SO_3H$, $-CO_2H$, -COO-$C_1$-$C_6$-Alkyl, CONH-$C_1$-$C_6$-Alkyl, CON($C_1$-$C_6$-Alkyl)$_2$, $-NHR_5$ oder $NR_5R_6$ bedeuten, wobei $R_5$ und $R_6$ die im Anspruch 1 angegebenen Bedeutungen haben.

Die folgenden Erläuterungen stellen wichtige beispielhafte Angaben dar:

Die in der Formel I vorkommenden Alkylgruppen, Halogenalkylgruppen, Alkenylgruppen, Alkinylgruppen, Alkoxygruppen, Alkylaminogruppen, Alkanoylaminogruppen, Alkanoyloxygruppen beziehungsweise ganz allgemein Alkanoylgruppen können gerade oder verzweigt sein. Dasselbe gilt auch für Alkyl- und Alkyloxygruppen (= Alkoxygruppen), falls diese Bestandteil anderer zusammengesetzter Gruppen sind (zum Beispiel in Form einer Monoalkyl- oder Dialkylaminogruppe, Alkanoylaminogruppe, Alkoxycarbonylaminogruppe, Carbalkoxygruppe und analoge Gruppen.

Bei der $C_3$-$C_7$-Cycloalkylgruppe handelt es sich vorzugsweise um Cyclopentyl oder Cyclohexyl. $C_2$-$C_6$-Alkenyl bedeutet vorzugsweise Propenyl. $C_2$-$C_6$-Alkinyl bedeutet vorzugsweise Propinyl.

Bei den Halogenatomen handelt es sich um Chlor, Brom oder Fluor, Insbesondere Chlor und Fluor. Die Alkyl- und Alkoxygruppen als solche oder als Bestandteil von anderen zusammengesetzten Gruppen bestehen insbesondere aus 1-4 C-Atomen, vorzugsweise 1 oder 2 C-Atomen. Alkanoylgruppen, wie zum Beispiel Alkanoylaminogruppen oder Alkanoyloxygruppen bestehen insbesondere aus 2-4, vorzugsweise 2-3 C-Atomen. Alk besteht insbesondere aus 1-4, vorzugsweise 2-3 C-Atomen.

Vorzugsweise handelt es sich bei den Verbindungen der Formel I um Verbindungen, wo die Reste $R_1$, $R_2$, $R_3$ und $R_4$ die folgenden Bedeutungen haben:

$R_1$: Wasserstoff, $C_1$-$C_6$-Alkyl oder die Gruppe

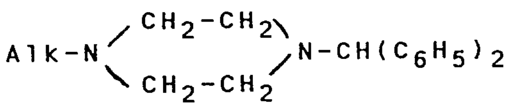

wobei einer oder beide Phenylreste auch durch Halogen, insbesondere Fluor substituiert sein können.

$R_2$: Wasserstoff, $C_1$-$C_6$-Alkoxy oder die Gruppe G-Alk-A, worin G Sauerstoff ist, Alk aus 2-4 C-Atomen besteht und A die Gruppe $CO$-$NR_5R_6$ ist mit $R_5$ = $C_1$-$C_4$-Alkyl und $R_6$ = $C_4$-$C_7$-Cycloalkyl.

Der Rest $R_2$ befindet sich vorzugsweise in 8-Stellung.

$R_3$ und $R_4$: Wasserstoff

Besonders günstige Eigenschaften haben beispielsweise folgende Verbindungen der Formel I:

D 21247 (Beispiel 1) , D 19897 (Beispiel 12),
D 20971 (Beispiel 28), D 20467,
D 20972 (Beispiel 29), D 20469,
D 20896 (Beispiel 11), D 20354.

D 20354 = 4,5-Dihydro-8-[2-hydroxy-3-(4-(2-methoxyphenyl)piperazin-1-yl)-propyloxyl-4-oxo-pyrido-[3,2-e]pyrrolo[1,2-a]pyrazinhydrochlorid

D 20467 = 4,5-Dihydro-8-[2-hydroxy-3-(4-benzhydrylpiperazin-1-yl)-propyloxy]-4-oxo-pyrido-[3,2-e]-pyrrolo[1,2-a]pyrazin-dihydrochloridmonohydrat

D 20469 = 4,5-Dihydro-8-[2-hydroxy-3-(4-phenylpiperidin-1-yl)-propyloxy]-4-oxo-pyrido-[3,2-e]-pyrrolo[1,2-a]pyrazin-hydrochlorid

Diejenigen Verbindungen der Formel I, die asymmetrische Kohlenstoffatome enthalten und in der Regel als Razemate anfallen, können in an sich bekannter Weise beispielsweise mit Hilfe einer optisch aktiven Säure in die optisch aktiven Isomeren gespalten werden. Es ist aber auch möglich, von vornherein eine optisch aktive Ausgangssubstanz einzusetzen, wobei dann als Endprodukt eine entsprechende optisch aktive beziehungsweise diastereomere Form erhalten wird. Die vorliegende Erfindung umfaßt also auch die D- und L-Form wie auch die DL-Mischung für den Fall, daß in der Verbindung der Formel I ein asymmetrisches C-Atom vorkommt und für den Fall von 2 und mehr asymmetrischen C-Atomen ebenso die entsprechenden diastereomeren Formen.

Je nach den Verfahrensbedingungen und Ausgangsstoffen erhält man die Endstoffe der Formel I in freier Form oder in Form ihrer Salze. Die Salze der Endstoffe können in an sich bekannter Weise, beispielsweise mit Alkali oder Ionenaustauschern wieder in die Basen übergeführt werden. Von den letzteren lassen sich durch Umsetzung mit organischen oder anorganischen Säuren, insbesondere solchen, die zur Bildung von therapeutisch verwendbaren Salzen geeignet sind, Salze gewinnen.

Die erfindungsgemäßen Verbindungen sind zur Herstellung pharmazeutischer Zusammensetzungen geeignet. Die pharmazeutischen Zusammensetzungen beziehungsweise Medikamente können eine oder mehrere der erfindungsgemäßen Verbindungen enthalten. Zur Herstellung der pharmazeutischen Zubereitungen können die üblichen Träger- und Hilfsstoffe verwendet werden.

Zu dem Verfahren gemäß Anspruch 2

Das Verfahren kann ohne Lösungsmittel oder in einem geeigneten Lösungs- oder Dispergiermittel durchgeführt werden. Als Lösungs- oder Dispergiermittel kommen zum Beispiel in Betracht: Aromatische Kohlenwasserstoffe wie zum Beispiel Benzol, Mesitylen, Toluol, Xylol; Pyridin; niedere aliphatische Ketone wie zum Beispiel Aceton, Methyläthylketon; halogenierte Kohlenwasserstoffe wie zum Beispiel Chloroform,

1,2-Dichlorethan, Tetrachlorkohlenstoff, Chlorbenzol, Methylenchlorid; Ether wie zum Beispiel Tetrahydrofuran, Dioxan, Diisopropylether; Sulfoxyde wie zum Beispiel Dimethylsulfoxid; tertiäre Säureamide wie zum Beispiel Diemthylformamid, Dimethylacetamid, Hexamethylphosphorsäuretriamid, Tetramethylharnstoff, N-Methylpyrrolidon; niedere Alkohole wie zum Beispiel Methanol, Äthanol, Isopropanol, Amylalkohol, Butanol, tert.-Butanol sowie Mischungen der genannten Mittel, gegebenenfalls auch mit Wasser.
Es empfiehlt sich die Reaktion unter einer Schutzgasatmosphäre durchzuführen. Als Schutzgase kommen beispielsweise in Frage: Stickstoff, Argon.

Die Reaktion wird beispielsweise bei Temperaturen zwischen 20 bis 200°C, vorzugsweise 40 bis 160°C oder auch 50 bis 120°C durchgeführt.
Wird ein Lösungs- beziehungsweise Dispersionsmittel verwendet, arbeitet man häufig bei der Rückflußtemperatur dieses Mittels. Die Reaktion läuft häufig bereits auch schon bei Raumtemperatur ab, beziehungsweise bei einer Temperatur zwischen 40 bis 120°C.
Bei der Ausgangskomponente $R_1$ Hal bedeutet Hal vorzugsweise Chlor, Brom oder Jod. Dies gilt auch für die Ausgangskomponente Hal-Alk-A.

Die Umsetzung wird vorteilhaft in Gegenwart von säurebindenden Mitteln wie Alkalicarbonaten (Pottasche, Soda), Alkaliacetaten, Alkalihydroxyden oder tertiären Basen (Triethylamin, Pyridin) durchgeführt.
Vorzugsweise wird die Ausgangsverbindung II in Form ihres Metallsalzes eingesetzt. Insbesondere kommen hier die Alkalisalze (Na, K, Li) in Frage.
Die Herstellung der Alkalisalze erfolgt beispielsweise mittels den entsprechenden Alkalihydriden, Alkaliamiden, Alkalialkoholaten oder auch Alkalimetallen in einem Lösungsmittel (niederer Alkohol, aromatischer Kohlenwasserstoff) oder mit wäßrigem Alkali (zum Beispiel NaOH).

Alkylierung, Acylierung und Aminierung

Insbesondere handelt es sich hier um die Acylierung oder Alkylierung von Aminogruppen und/oder Hydroxygruppen oder um den Ersatz eines Halogenatoms durch $NH_2$ beziehungsweise alkylsubstituiertes Amino. Die Alkylierung beziehungsweise Acylierung erfolgt beispielsweise durch Umsetzung mit Verbindungen der Formel R-Hal, $ArSO_2OR$ und $SO_2(OR)_2$, wobei Hal ein Halogenatom (insbesondere Chlor, Brom oder Jod) und Ar ein aromatischer Rest (zum Beispiel ein gegebenenfalls durch einen oder mehrere niedere Alkylreste substituierter Phenyl- oder Naphthylrest ist und R beispielsweise die Reste $C_1$-$C_6$-Alkyl, $C_2$-$C_6$-Alkenyl, $C_2$-$C_6$-Alkinyl , $C_2$-$C_6$-Alkanoyl, Benzoyl, Morpholinocarbonyl, $C_1$-$C_6$-Alkyloxycarbonyl, $C_1$-$C_6$-Alkylaminocarbonyl, $C_1$-$C_6$-Dialkylaminocarbonyl, die Gruppe COD (Bedeutungen von D wie im Patentanspruch angegeben) oder Ar-$C_1$-$C_6$-Alkyl bedeutet.

Beispiele sind entsprechende p-Toluolsulfonsäure-alkylester, entsprechende Dialkylsulfate, entsprechende Alkylhalogenide und ähnliche. Die Alkylierungs-und Acylierungsreaktion wird gegebenenfalls unter Zusatz von üblichen säurebindenden Mitteln, wie Alkalihydroxiden, Alkalicarbonaten, Alkalihydrocarbonaten, Alkalihydrogencarbonaten, Erdalkalicarbonaten, Erdalkaliacetaten, tertiaren Aminen (zum Beispiel Trialkylamin wie Triethylamin), Pyridin oder auch Alkalihydriden bei Temperaturen zwischen 0 und 200°C, vorzugsweise 40 und 140°C in inerten Lösungsmitteln oder Suspensionmitteln durchgeführt. Als Lösungs- oder Dispergiermittel kommen beispielsweise in Betracht: aromatische Kohlenwasserstoffe wie zum Beispiel Benzol, Toluol, Xylol; aliphatische Ketone wie zum Beispiel Aceton, Methylethylketon; halogenierte Kohlenwasserstoffe wie zum Beispiel Chloroform, Tetrachlorkohlenstoff, Chlorbenzol, Methylenchlorid, aliphatische Ether wie zum Beispiel Butylether; cyclische Ether wie zum Beispiel Tetrahydrofuran, Dioxan; Sulfoxyde wie zum Beispiel Dimethylsulfoxid; tertiäre Säureamide wie zum Beispiel Dimethylformamid, N-Methylpyrrolidon, Hexamethylphosphorsäuretriamid; aliphatische Alkohole wie Methanol, Ethanol, Isopropanol, Amylalkohol, tert.-Butanol, cycloaliphatische Kohlenwasserstoffe wie Cyclohexan und ähnliche. Auch wässrige Mischungen der genannten Lösungsmittel können verwendet werden. Häufig arbeitet man bei der Rückflußtemperatur der verwendeten Lösungs- beziehungsweise Dispergiermittel. Häufig werden die Alkylierungsreaktionskomponenten im Überschuß eingesetzt. Die Alkylierung kann auch in Gegenwart von Tetraalkylammoniumsalzen (insbesondere der Halogenide) in Kombination mit Alkalihydroxiden bei Temperaturen zwischen 0 - 100°C, vorzugsweise 20 -80°C, in einem aprotischen Lösungsmittel oder auch in Chloroform oder Methylenchlorid vorgenommen werden. Als aprotische Lösungsmittel kommen insbesondere in Betracht: tertiäre Amide (Dimethylformamid, N-Methyl-Pyrrolidon, Hexamethylphosphorsäuretriamid), Dimethylsulfoxid, Acetonitril, Dimethoxyethan, Aceton, Tetrahydrofuran.

Bei der Acylierung werden zum Beispiel in Aminogruppen oder Hydroxygruppen die Gruppe -COD (Bedeutungen von D wie im Anspruch angegeben), die Gruppe -CO-$C_1$-$C_6$-Alkyl, Benzoyl oder die $C_1$-$C_6$-Alkoxycarbonylgruppe eingeführt.
Man verfährt hierbei in an sich bekannter weise beispielsweise verwendet man die entsprechenden

Säurehalogenide (Chloride, Bromide) wie zum Beispiel Carb-$C_1$-$C_6$-alkoxyhalogenide oder entsprechende Anhydride). Die Reaktionstemperaturen liegen vorzugsweise zwischen 30 und 120°C.

Gegebenenfalls kann man bei der Alkylierung und der Acylierung auch so vorgehen, daß man zuerst von der zu alkylierenden beziehungsweise acylierenden Verbindung eine Alkaliverbindung (Natrium-, Kalium- oder auch Lithiumsalz zum Beispiel) herstellt, indem man sie in einem inerten Lösungsmittel wie Dioxan, Dimethylformamid, Benzol oder Toluol mit einem Alkalimetall, Alkalihydrid oder Alkaliamiden (insbesondere Natrium oder Natriumverbindungen) oder Butyllithium bei Temperaturen zwischen 0 und 150°C umsetzt und dann das alkylierden Agens zufügt.

Anstelle der angeführten Alkylierungs- und Acylierungsmittel können auch andere in der Chemie gebräuchliche chemisch-äquivalente Mittel verwendet werden (siehe zum Beispiel auch L.F. und Mary Fieser "Reagents for Organic Synthesis", John wiley and Sons, Inc., New York, 1967, Vol. 1, Seitten 1303-4 und Vol. 2, Seite 471.

Vorhandene Acylgruppen wie zum Beispiel Carb-$C_1$-$C_6$-alkoxygruppen, $C_2$-$C_6$-Alkanoylgruppen und ähnliche Gruppen können solvolytisch abgespalten werden. Diese Abspaltung erfolgt in bekannter weise beispielsweise durch Verseifung mit Säuren (Mineralsäuren wie Salzsäure, Schwefelsäure, insbesondere konzentrierten Halogenwasserstoffsäuren wie HBr/Eisessig) oder mittels basischer Substanzen (Pottasche, Soda, wässrige Alkalilösungen, alkoholische Alkalilösungen, wässriges $NH_3$) bei Temperaturen zwischen 10 und 150°C, insbesondere 20 - 100°C.

Bei der Aminierung handelt es sich um die Umsetzung einer Halogenalkylfunktion (zum Beispiel $R_1$ ist Alk-A und/oder $R_2$, $R_3$ sind G-AlkA, wo A ein Halogenatom wie Chlor, Brom oder Jod ist) mit $-NHR_5$, $-NR_5R_6$, $NR_5R_6R_7$, Pyrdyilamino, Imidazolyl, Pyrrolidinyl, N-$C_1$-$C_6$-Alkylpyrrolidinyl, Piperidylamino, N-(Phenyl-$C_1$-$C_4$-alkyl)-piperidylamino, wobei $R_5$ und $R_6$ gleich oder verschieden sind und Wasserstoff, $C_1$-$C_6$-Alkyl, $C_3$-$C_7$-Cycloalkyl, $C_3$-$C_7$-Hydroxycycloalkyl, Morpholino-$C_1$-$C_6$-Alkyl, Phenyl, Phenyl-$C_1$-$C_6$-alkyl oder Phenyl-$C_2$-$C_6$-oxyalkyl darstellen, wobei die Phenylreste auch durch Halogen substituiert sein können und $R_7$ wasserstoff oder $C_1$-$C_6$-Alkyl ist, oder um die Umsetzung einer solchen Halogenalkylfunktion mit Aminen der folgenden Formel

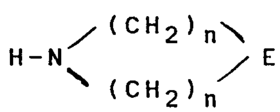

worin n die Zahlen 1-3 annehmen kann und E $CH_2$, Sauerstoff, Schwefel, NH, CHOH, CH-$C_1$-$C_6$-Alkyloxy, CH-$C_2$-$C_6$-Alkanoyloxy, $CHC_6H_5$, CHCOD, $CH$-$CH_2C_6H_5$, N-$C_1$-$C_6$-Alkyl, N-$C_1$-$C_6$-Hydroxyalkyl, N-$C_6H_5$, N-$CH_2C_6H_5$, N-$CH(C_6H_5)2$, N-$(CH_2)_2$-OH, N-$(CH_2)_3$-OH oder NCOD darstellt und die Phenylreste ($C_6H_5$) auch durch Halogen, $C_1$-$C_6$-Alkoxy, Trifluormethyl, $C_1$-$C_6$-Alkyl, Methylendioxy, Cyan substituiert sein können und D Phenyl, $C_1$-$C_6$-Alkyl, $C_3$-$C_7$-Cycloalkyl, Hydroxy, $C_1$-$C_6$-Alkoxy, $C_3$-$C_7$-Cycloalkyloxy, Morpholino, Pyrrolidino, Piperidino, Homopiperidino, Piperazino, $-NHR_5$ oder $-NR_5R_6$ ist und $R_5$ und $R_6$ die angegebenen Bedeutungen haben.

Diese Umsetzung erfolgt gemäß den Bedingungen, die für das Verfahren gemäß Patentanspruch 2 angegeben sind.

Die Abspaltung von Ethergruppen erfolgt beispielsweise ohne Lösungsmittel oder in einem inerten Lösungsmittel mit Bortribromid, Bortrifluorid, Aluminiumchlorid, Siliziumtetrachlorid, Aluminiumtribromid, Natriumethylthiolat, $(CH_3)_3SiCl + NaJ$ bei Temperaturen Zwischen -70°C und 200°C. Als Lösungsmittel für diese Etherspaltung kommen beispielsweise in Frage: aliphatische Halogenkohlenwasserstoffe wie zum Beispiel Methylenchlorid, aromatische Kohlenwasserstoffe wie Benzol, Toluol, Xylol, halogenierte aromatische Kohlenwasserstoffe wie Chlorbenzol, Dichlorbenzole, Dimethylformamid, Acetonitril.

Weiterhin kann diese Etherspaltung auch mittels Jod-Wasserstoffsäure, Chlorwasserstoffsäure (zum Beispiel alkoholische HCl), Pyridinhydrochlorid, Bromwasserstoffsaure, Methylmagnesiumjodid mit oder ohne Lösungsmittel bei Temperaturen zwischen 0°C bis 150°C erfolgen. Als Lösungsmittel für die zuletzt genannte Spaltung kommen beispielsweise in Betracht: aliphatische Ether mit Alkylresten aus 1 bis 6 C-Atomen, Wasser.

Einzelheiten über die Reaktionsbedingungen und Reaktionskomponenten der vorstehend genannten Umsetzungen gehen auch aus der Herstellungsbeschreibung für die Ausgangsstoffe hervor.

Die Umsetzung einer Verbindung III mit der Verbindung IV erfolgt in einem Lösungs- beziehungsweise Suspensionsmittel (niedere aliphatische Alkohole, Dimethylsulfoxid, Wasser und wässrige Gemische) bei Temperaturen zwischen 10 und 150°C, vorzugsweise 20 - 80°C. Zweckmäßig wird in Gegenwart basischer

Stoffe wie Alkalikarbonaten, tertiären aliphatischen Aminen, Pyridin gearbeitet.

Herstellung der Verbindung VI durch reduktive Behandlung der Verbindung V

Für diese Reduktion hat sich als besonders geeignet die katalytische Hydrierung erwiesen. Als Katalysatoren kommen zum Beispiel in Frage: Raney-Nickel, Edelmetalle wie Palladium und Platin sowie Verbindungen davon, mit und ohne Träger, wie beispielsweise Kohle, Bariumsulfat, Calciumsulfat und so weiter. Es empfiehlt sich, die Hydrierung der Nitrogruppe bei Temperaturen zwischen 20 und 100°C und einem Druck von ungefähr 1 bis 70 bar in einem Lösungsmittel vorzunehmen. Als Lösungsmittel eignen sich beispielsweise $C_1$-$C_4$-Alkanole, $C_2$-$C_4$-Diole und deren Niederalkylether, cyclische Ether wie Dioxan, Tetrahydrofuran, Methoxy-ethanol, Wasser, aromatische Kohlenwasserstoffe (Benzol, Toluole, Xylole) sowie Gemische dieser Mittel. Für die anschließende Isolierung der reduzierten Verbindungen kann es in manchen Fällen von Vorteil sein, wenn zu Beginn dem zu hydrierenden Gemisch Trockenmittel, wie wasserfreies Natrium- oder Magnesiumsulfat zugesetzt werden.

Die Reduktion kann aber auch mit nascierendem Wasserstoff beispielsweise Zink/Salzsäure, Zinn/Salzsäure, Eisen/Salzsäure, Eisen/Eisessig oder mit Salzen des Schwefelwasserstoffs in Alkohol/Wasser bei etwa 70 bis etwa 120°C oder mit aktiviertem Aluminium in wasserhaltigem Äther bei 20 bis 40°C oder mit ZinnII-Chlorid/Salzsäure oder mit Ammoniumformiat durchgeführt werden.

Detaillierte Erläuterung des neuen erfindungsgemäßen Verfahrens gemäß Anspruch 3

Allgemeine Synthesevorschrift zur Darstellung von N-Aryl- oder N-Pyridyl-2-ethoxycarbonyl-4-hydroxypyrrolidinen (Verbindungen der Formel V)

Verfahrensvariante A:

Eine Mischung aus 1 mol der entsprechenden o-Halogennitroaromaten, 234,8 g (1,2 mol) L-4-Hydroxyprolinethylesterhydrochlorid und 414.6 g (3 mol) Kaliumcarbonat in 2 Liter Ethanol wird unter Rühren 20-120 h auf 50-60°C erwärmt. Nach Abkühlen auf Raumtemperatur filtriert man von unlöslichen Bestandteilen ab und engt das Reaktionsgemisch im Vakuum ein. Den verbleibenden öligen Rückstand nimmt man in 1l Dichlormethan auf und extrahiert je einmal mit 400 ml halbkonzentrierter Salzsäure und 200 ml Wasser. Die organische Phase wird mit Calciumchlorid getrocknet und anschließend im Vakuum eingeengt. Nach Trocknen im Vakuum erhält man in 65-95prozentiger Ausbeute die entsprechenden N-Aryl- oder N-Pyridyl-2-ethoxycarbonyl-4-hydroxypyrrolidine, die in der Regel ohne weitere Aufreinigung in die nächste Verfahrensstufe eingesetzt werden.

Verfahrensvariante B:

Eine Mischung aus 1 mol der entsprechenden o-Halogennitroaromaten, 157,4 g (1,2 mol) L-4-Hydroxyprolin und 202,4 g (2 mol) Triethylamin in 1,5 l Dimethylsulfoxid wird unter Rühren 20-120 h auf 50-70°C erwärmt. Nach Abkühlen auf Raumtemperatur wird das Reaktionsgemisch mit 4,5 l Wasser versetzt und die resultierende Lösung zweimal mit je 500 ml Ether extrahiert. Der Etherextrakt wird verworfen und die wässrige Phase mit konzentrierter Salzsäure auf pH = 2-3 eingestellt. Die wässrige Phase wird daraufhin viermal mit je 750 ml Dichlormethan extrahiert. Die vereinigten Dichlormethanextrakte werden mit Natriumsulfat getrocknet und anschließend im Vakuum eingeengt. Nach Trocknen im Vakuum erhält man in 65-95prozentiger Ausbeute die entsprechenden N-Aryl- oder N-Pyridyl-2-carboxy-4-hydroxypyrrolidine, die in der Regel ohne weitere Aufreinigung in die nächste Verfahrensstufe eingesetzt werden.

Nach Variante A wurden beispielsweise hergestellt:

2-[1-(2-Ethoxycarbonyl-4-hydroxy)pyrrolidinyl]-6-methoxy-3-nitropyridin: orangegelbes Öl.

1-[1-(2-Ethoxycarbonyl-4-hydroxy)pyrrolidinyl]-2-nitrobenzol: orangegelbes Öl.

Allgemeine Synthesevorschrift zur Herstellung annellierter Pyrrolo[1,2-a]-pyrazinone (Verbindungen der Formel VI) und deren Oxydation zu Verbindungen der Formel VII

Verfahrensvariante A

Eine Lösung aus 0,5 mol der entsprechenden o-[1-(2-Ethoxycarbonyl-4-hydroxy)-pyrrolidinyl]-nitroaromaten beziehungsweise o-[1-(2-carboxy-4-hydroxy)-pyrrolidinyl]-nitroaromaten in 1,5 l Methanol wird in Gegenwart von Palladium auf Aktivkohle unter Normaldruck bei ca. 60°C hydriert. Nach beendeter

Wasserstoffaufnahme filtriert man den Katalysator ab, verdünnt das Filtrat mit 3 l Methanol, säuert mit methanolischer Salzsäure an (pH = 1-2) und rührt die resultierende Lösung unter Durchleiten von Luft 1-5 Tage bei Raumtemperatur. Man engt die Lösung im Vakuum auf ca. 300 ml ein und läßt über Nacht bei 0-4°C kristalltsieren. Die ausgefallenen Kristalle werden abgesaugt, mit 100 ml Methanol nachgewaschen und im Vakuum getrocknet.

Verfahrensvariante B

Eine Lösung aus 0,5 mol der entsprechenden o-[1-(2-Ethoxycarbonyl-4-hydroxy)-pyrrolidinyl]-nitroaromaten beziehungsweise o-[1-(2-carboxy-4-hydroxy)-pyrrolidinyl]-nitroaromaten in 1 l Eisessig wird unter Rühren und Stickstoffatmosphäre portionsweise mit 75 g Eisenpulver versetzt. Nach beendeter Zugabe erwärmt man das Reaktionsgemisch 3-5 h auf 60-70°C. Nach Abkühlen auf Raumtemperatur filtriert man von unlöslichen Bestandteilen ab und engt das Filtrat im Vakuum bis zur Trockene ein. Der verbleibende Rückstand wird erschöpfend mit Dichlormethan extrahiert. Die Methylenchloridphase wird im Vakuum eingeengt, der Rückstand in 3 l Methanol aufgenommen und die resultierende Lösung mit methanolischer Salzsäure angesäuert (pH = 1-2). Anschließend rührt man 1-5 Tage unter Durchleiten von Luft bei Raumtemperatur. Die Reaktionslösung wird auf ca. 300 ml eingeengt und über Nacht bei 0-4 °C zur Kristallisation gebracht. Die Kristalle werden abgesaugt, mit Methanol nachgewaschen und im Vakuum getrocknet.

Nach Variante A wurden beispielsweise folgende Verbindungen erhalten:
4,5-Dihydro-8-methoxy-4-oxo-pyrido[3,2-e]pyrrolo[1,2-a]pyrazin (Chiffre D 17500).
Farblose bis beige Kristalle, F. 274-276 °C.
4,5-Dihydro-4-oxo-pyrrolo[1,2-a]chinoxalin (Chiffre D 18543).
Farblose bis beige Kristalle, F. 271-273°C.
4,5-Dihydro-4-oxo-pyrido[3,2-e]pyrrolo[1,2-alpyrazin (Chiffre D 18367).
Farblose bis beige Kristalle, F. 281-284°C.
8-Chlor-4,5-dihydro-4-oxo-pyrido[3,2-e]pyrrolo[1,2-a]pyrazin (Chiffre D 18370).
Farblose bis beige Kristalle, F. 297-299°C.
4,5-Dihydro-8-methyl-4-oxo-pyrrolo[1,2-a]chinoxalin (Chiffre D 19786).
Farblose bis beige Kristalle, F. 283-285°C.

Nach Variante B wurden beispielsweise erhalten:
4,5-Dihydro-7-methyl-4-oxo-pyrrolo[1,2-a]chinoxalin (Chiffre D 19777).
Farblose bis beige Kristalle, F. 258 °C.
7-Amino-4,5-dihydro-4-oxo-pyrrolo[1,2-a]chinoxalinhydrochlorid (Chiffre D 19457).
Dunkelbeige Kristalle, F. 324-328°C.
4,5-Dihydro-7-trifluormethyl-4-oxo-pyrrolo[1,2-a]-chinoxalin (Chiffre D 19801).
Farblose bis beige Kristalle, F. 240-241°C
4,5-Dihydro-8-fluor-4-oxo-pyrrolo[1,2-a]chinoxalin (Chiffre D 19823).
Farblose bis beige Kristalle, F. 318°C.
4,5-Dihydro-7-fluor-4-oxo-pyrrolo[1,2-a]chinoxalin (Chiffre D 19857).
Farblose bis beige Kristalle, F. 281 °C.
9-Chlor-4,5-dihydro-4-oxo-pyrrolo[1,2-a]chinoxalin (Chiffre D 19858).
Farblose bis beige Kristalle, F. 309°C.
7-Cyano-4,5-dihydro-4-oxo-pyrrolo[1,2-a]chinoxalin (Chiffre D 20353)
Farblose bis beige Kristalle, F. 312°C.

Die Ausgangssubstanzen für die Herstellung der Verbindungen gemäß Anspruch 1 sind bekannt oder können nach dem Verfahren gemäß Anspruch 3 und/oder analog den folgenden Beispielen hergestellt werden.

Beispiel I

4,5-Dihydro-8-hydroxy-4-oxo-pyrido[3,2-e]pyrrolo[1,2-alpyrazin (Chiffre D 19459).
Eine Mischung aus 107,6 g (0,5 mol) 4,5-Dihydro-8-methoxy-4-oxo-pyrido[3,2-e]pyrrolo[1,2-a]pyrazin und 2 l 48prozentige wässrige Bromwasserstoffsäure wird unter Rühren und Schutzgasatmosphäre 5 h zum Rückfluß erhitzt. Nach Abkühlen auf Raumtemperatur saugt man das ausgefallene Produkt ab und wäscht mit 500 ml Wasser nach. Der Feststoff wird in 1000 ml Wasser aufgenommen und die resultierende Suspension unter Rühren mit konzentriertem Ammoniak alkalisch gestellt (pH ≧ 10). Das Lösungsmittel wird abfiltriert und der Filterkuchen in 1000 ml Ethanol unter Rühren 1 h zum Rückfluß erhitzt. Nach dem

Erkalten saugt man den Feststoff ab, wäscht mit 200 ml Ethanol nach und trocknet im Vakkuum. Man erhält 87,9 g 4,5-Dihydro-8-hydroxy-4-oxo-pyrido[3,2-e]pyrrolo[1,2-a]pyrazin als beigefarbene Kristalle, F. 364-368°C.

Beispiel II

4,5-Dihydro-8-[2-hydroxy-3-(4-(2-methoxyphenyl)-piperazin-1-yl)-propyloxy]-4-oxo-pyrido[3,2-e]-pyrrolo-[1,2-a]pyrazin-hydrochlorid (Chiffre D 20354). Eine Suspension von 4,0 g (20 mmol) 4,5-Dihydro-8-hydroxy-4-oxo-pyrido[3,2-e]pyrrolo[1,2-a]pyrazin in 65 ml Dimethylformamid wird unter Rühren und Feuchtigkeitsausschluß portionsweise mit 0,8 g (20 mmol) 80prozentigem Natriumhydrid in Weißöl versetzt. Nach beendeter Zugabe rührt man 2 h bei Raumtemperatur nach und tropft anschließend unter Rühren und Schutzgasatmosphäre eine Lösung von 5,7 g (20 mmol) 1-(3-Chlor-2-hydroxypropyl)-4-(2-methoxyphenyl)-piperazin in 30 ml Dimethylformamid zu. Nach beendeter Zugabe erwärmt man allmählich auf ca. 100-110°C und läßt 3 h bei dieser Temperatur nachreagieren. Nach Abkühlen auf Raumtemperatur filtriert man von unlöslichen Bestandteilen ab und engt das Filtrat im Vakkuum bis zur Trockene ein. Der verbleibende Rückstand wird in Ethanol aufgenommen und die nach Filtration resultierende Lösung mit ethanolischer Salzsäure angesäuert (pH ≦ 2). Der ausgefallene Feststoff wird abgesaugt und aus Ethanol/Wasser-Gemisch umkristallisiert. Nach Trocknen im Vakuum erhält man 3,8 g der oben angegebenen Verbindung D 20354 als beigefarbene Kristalle, F. 251-255°C.

Analog werden folgende Verbindungen erhalten:

4,5-Dihydro-8-[2-(4-(2-fluorphenylpiperazin-1-yl) ethoxy]-4-oxo-pyrido[3,2-e]pyrrolo[1,2-a]pyrazinhydrochlorid (Chiffre D 20366).

4,0 g (20 mmol) 4,5-Dihydro-8-hydroxy-4-oxo-pyrido[3,2-e]pyrrolo[1,2-a]pyrazin werden analog Beispiel II mit 4,6 g (20 mmol) 1-(2-Chlorethyl)-4-(2-fluorphenyl)piperazin umgesetzt und entsprechend aufgearbeitet. Man erhält 1,5 g der Substanz D 20366 als beigefarbene Kristalle, F. > 260°C.

8-[3-(4-(3-Chlorphenylpiperazin-1-yl)-propyloxy-4,5-dihydro-4-oxo-pyrido[3,2-e]pyrrolo[1,2-a]-pyrazinhydrochlorid (Chiffre D 20 367).

4,0 g (20 mmol) 4,5-Dihydro-8-hydroxy-4-oxo-pyrido[3,2-e]pyrrolo[1,2-a]pyrazin werden analog Beispiel II mit 5,5 g (20 mmol) 1-(3-Chlorpropyl)-4-(3-chlorphenyl)piperazin umgesetzt und entsprechend aufgearbeitet. Man erhält 4,1 g der Substanz D 20367 als beigefarbene Kristalle, F. > 260°C.

4,5-Dihydro-8-[3-(4-(2-fluorphenylpiperazin-1-yl)-propyloxy-4-oxo-pyrido-[3,2-e]pyrrolo[1,2-a]-pyrazinhydrochlorid (Chiffre D 20368).

4,0 g (20 mmol) 4,5-Dihydro-8-hydroxy-4-oxo-pyrido-[3,2-e]pyrrolo[1,2-a]pyrazin werden analog Beispiel II mit 5,1 g (20 mmol ) 1-(3-Chlorpropyl)-4-(2-fluorphenyl)piperazin umgesetzt und entsprechend aufgearbeitet. Man erhält 2,1 g der Substanz D 20368 als beigefarbene Kristalle, F > 260°C.

4,5-Dihydro-8-[2-(4-(2-nitrophenylpiperazin-1-yl)-ethoxy]-4-oxo-pyrido[3,2-e]pyrrolo[1,2-a]-pyrazinhydrochlorid (Chiffre D 20369).

4,0 g (20 mmol) 4,5-Dihydro-8-hydroxy-4-oxo-pyrido[3,2-e]pyrrolo[1,2-a]pyrazin werden analog Beispiel II mit 5,4 g (20 mmol) 1-(2-Chlorethyl)-4-(2-nitrophenyl)piperazin umgesetzt und entsprechend aufgearbeitet. Man erhält 2,4 g der Substanz D 20369 als beigefarbene Kristalle, F > 260°C.

4,5-Dihydro-8-[2-hydroxy-3-(4-phenylpiperazin-1-yl)-propyloxy]-4-oxo-pyrido[3,2-e]pyrrolo[1,2-a]-pyrazinhydrochlorid (Chiffre D 20381).

4,0 g (20 mmol) 4,5-Dihydro-8-hydroxy-4-oxo-pyrido[3,2-e]pyrrolo[1,2-a]pyrazin werden analog Beispiel II mit 5,1 g (20 mmol) 1-(2-Chlorethyl)-4-phenyl)piperazin umgesetzt und entsprechend aufgearbeitet. Man erhält 1,7 g der Substanz D 20381 als beigefarbene Kristalle, F > 260°C.

4,5-Dihydro-8-[2-(4-phenylpiperazin-1-yl)-ethoxy]-4-oxo-pyrido[3,2-e]pyrrolo[1,2-a]pyrazinhydrochlorid-monohydrat (Chiffre D 20391).

4,0 g (20 mmol 4,5-Dihydro-8-hydroxy-4-oxo-pyrido[3,2-e]pyrrolo[1,2-alpyrazin werden analog Beispiel II mit 4,5 g (20 mmol) 1-(2-Chlorethyl)-4-phenyl-piperazin umgesetzt und entsprechend aufgearbeitet. Man erhält 2,2 g der Substanz D 20391 als beigefarbene Kristalle, F > 260°C.

4,5-Dihydro-8-[2-(4-(3-chlorphenyl)piperazin-1-yl)-ethoxy[-4-oxo-pyrido[3,2-e]pyrrolo[1,2-a]-pyrazinhydrochlorid-monohydrat (Chiffre D 20408).

4,0 g (20 mmol) 4,5-Dihydro-8-hydroxy-4-oxo-pyrido[3,2-e]pyrrolo[1,2-a]pyrazin werden analog Beispiel II mit 4,9 g (20 mmol) 1-(2-Chlorethyl)-4-(3-Chlorphenyl)-piperazin umgesetzt und entsprechend aufgearbeitet. Man erhält 2,9 g der Substanz D 20408 als beigefarbene Kristalle, F > 260°C.

4,5-Dihydro-8-[2-(4-(2-aminophenyl)piperazin-1-yl)-ethoxy]-4-oxo-pyrido[3,2-e]pyrrolo[1,2-a]-pyrazindihydrochlorid-monohydrat (Chiffre D 20409).

4,0 g (20 mmol) 4,5-Dihydro-8-hydroxy-4-oxo-pyrido[3,2-e]pyrrolo[1,2-a]pyrazin werden analog Beispiel II

mit 4,2 g (20 mmol) 1-(2-Chlorethyl)-4-(2-aminophenyl)-piperazin umgesetzt und entsprechend aufgearbeitet. Man erhält 1,5 g der Substanz D 20409 als beigefarbene Kristalle, F > 260°C.

4,5-Dihydro-8-[2-hydroxy-3-(4-(2-methylphenyl)-piperazin-1-yl)-propyloxy]-4-oxo-pyrido[3,2-e]-pyrrolo-[1,2-a]-pyrazin-hydrochlorid (Chiffre D 20410).

4,0 g (20 mmol) 4,5-Dihydro-8-hydroxy-4-oxo-pyrido[3,2-e]pyrrolo[1,2-a]pyrazin werden analog Beispiel II mit 5,4 g (20 mmol) 1-(3-Chlor-2-hydroxypropyl)-4-(2-methylphenyl)-piperazin umgesetzt und entsprechend aufgearbeitet. Man erhält 1,7 g der Substanz D 20410 als beigefarbene Kristalle, F > 260°C.

4,5-Dihydro-8-[3-(4-benzylpiperazin-1-yl)-propyloxy]-4-oxo-pyrido[3,2-e]pyrrolo[1,2-a]pyrazinhydrochlorid (Chiffre D 20426).

4,0 g (20 mmol) 4,5-Dihydro-8-hydroxy-4-oxo-pyrido[3,2-e]pyrrolo[1,2-a]pyrazin werden analog Beispiel II mit 4,5 g (20 mmol) 1-(3-Chlorpropyl)-4-benzylpiperazin umgesetzt und entsprechend aufgearbeitet. Man erhält 2,3 g der Substanz D 20426 als beigefarbene Kristalle, F > 260°C.

4,5-Dihydro-8-[2-hydroxy-3-(4-benzhydrylpiperazin-1-yl)-propyloxy]-4-oxo-pyrido[3,2-e]pyrrolo[1,2-a]-pyrazin-dihydrochlorid-monohydrat (Chiffre D 20467).

4,0 g (20 mmol) 4,5-Dihydro-8-hydroxy-4-oxo-pyrido[3,2-e]pyrrolo[1,2-a]pyrazin werden analog Beispiel II mit 6,9 g (20 mmol) 4-Benzhydryl-1-(3-chlor-2-hydroxypropyl)-piperazin umgesetzt und entsprechend aufgearbeitet. Man erhält 3,8 g der Substanz D 20467 als beigefarbene Kristalle, F > 260°C.

4,5-Dihydro-8-[2-hydroxy-3-(4-benzylpiperazin-1-yl)-propyloxy]-4-oxo-pyrido[3,2-e]pyrrolo[1,2-a]-pyrazin-dihydrochlorid-monohydrat (Chiffre D 20468).

4,0 g (20 mmol) 4,5-Dihydro-8-hydroxy-4-oxo-pyrido[3,2-e]pyrrolo[1,2-a]pyrazin werden analog Beispiel II mit 4,8 g (20 mmol) 4-Benzyl-1-(3-chlor-2-hydroxypropyl)-piperazin umgesetzt und entsprechend aufgearbeitet. Man erhält 2,6 g der Substanz D 20468 als beigefarbene Kristalle, F > 260°C.

4,5-Dihydro-8-[2-hydroxy-3-(4-phenylpiperidin-1-yl)-propyloxy]-4-oxo-pyrido[3,2-e]pyrrolo[1,2-a]-pyrazinhydrochlorid (Chiffre D 20469).

4,0 g (20 mmol) 4,5-Dihydro-8-hydroxy-4-oxo-pyrido[3,2-e]-pyrrolo[1,2-a]pyrazin werden analog Beispiel II mit 5,1 g (20 mmol) 1-(3-Chlor-2-hydroxypropyl)-4-phenylpiperidin umgesetzt und entsprechend aufgearbeitet. Man erhält 3,0 g der Substanz D 20469 als beigefarbene Kristalle, F > 260°C.

4,5-Dihydro-8-[2-(4-(2-methoxyphenyl)piperazin-1-yl)-ethoxy]-4-oxo-pyrido[3,2-e]pyrrolo[1,2-a]-pyrazinhydrochlorid (Chiffre D 20214).

4,0 g (20 mmol) 4,5-Dihydro-8-hydroxy-4-oxo-pyrido[3,2-e]-pyrrolo[1,2-a]pyrazin werden analog Beispiel II mit 5,1 g (20 mmol) 1-(2-Chlorethyl)-4-(2-methoxyphenyl)-piperazin umgesetzt und entsprechend aufgearbeitet. Man erhält 3,2 g der Substanz D 20214 als beigefarbene Kristalle, F. 260-262 °C.

4,5-Dihydro-8-[3-(4-(2-methoxyphenyl)piperazin-1-yl)-propyloxy]-4-oxo-pyrido[3,2-e]pyrrolo[1,2-a]-pyrazinhydrochlorid (Chiffre D 20215).

4,0 g (20 mmol) 4,5-Dihydro-8-hydroxy-4-oxo-pyrido[3,2-e]-pyrrolo[1,2-a]pyrazin werden analog Beispiel 11 mit 5,4 g (20 mmol) 1-(3-Chlorpropyl)-4-(2-methoxyphenyl)-piperazin umgesetzt und entsprechend aufgearbeitet. Man erhält 4,1 g der Substanz D 20215 als beigefarbene Kristalle, F. 265-267 °C.

4,5-Dihydro-8-[ethoxycarbonyl-methoxy]-4-oxo-pyrido[3,2-e]pyrrolo[1,2-a]pyrazin (Chiffre D 19919).

4,0 g (20 mmol) 4,5-Dihydro-8-hydroxy-4-oxo-pyrido[3,2-e]-pyrrolo[1,2-a]pyrazin werden analog Beispiel II mit 2,5 g (20 mmol) Chloressigsäureethylester umgesetzt und entsprechend aufgearbeitet. Man erhält 1,8 g der Substanz D 19919 als farblose Kristalle, F. > 198-199 °C.

4,5-Dihydro-8-[3-ethoxycarbonyl-butyloxy]-4-oxo-pyrido[3,2-e]-pyrrolo[1,2-a]pyrazin (Chiffre D 19937).

4,0 g (20 mmol) 4,5-Dihydro-8-hydroxy-4-oxo-pyrido[3,2-e]-pyrrolo[1,2-a]pyrazin werden analog Beispiel II mit 3,9 g (20 mmol) 5-Bromvaleriansäureethylester umgesetzt und entsprechend aufgearbeitet. Man erhält 2,8 g der Substanz D 19937 als farblose Kristalle, F. > 147-149°C.

Die erfindungsgemäßen Verbindungen zeigen in-vivo in verschiedenen Asthma-Modellen und in-vitro in der Inhibition von Mediatorfreisetzung eine gute antiallergische, antiasthmatische und antiinflammatorische Wirkung, sowie eine gute Wirkung auch beispielsweise bei Psoriasis und hiermit verwandten Eigenschaften.

Die niedrigste, bereits wirksame Dosis in dem oben angegebenen Tierversuch ist beispielsweise

10 mg/kg oral
0,1 mg/kg intravenös
1 mg/ml inhalativ

Als allgemeiner Dosisbereich für die
Wirkung (Tierversuch wie oben) kommt beispielsweise in Frage:

10-100 mg/kg oral, insbesondere 20-50 mg/kg
0,1-10 mg/kg intravenös, insbesondere 0,3-0,5 mg/kg
1 - 30 mg/ml inhalativ, insbesondere 5-15 mg/ml

Die Wirkungsrichtung der erfindungsgemäßen Verbindungen ist mit der Wirkung des bekannten Arzneimittelwirkstoffs DNCG vergleichbar, jedoch bestehen hierzu insbesondere folgende Unterschiede: potenter, es werden auch andere Mediatoren, nicht nur Histamin, inhibiert.

Indikationen für die die erfindungsgemäßen Verbindungen in Betracht kommen können: Asthma, allergische Rhinitis, rheumatische Arthritis, Psoriasis, Morbus Crohn, Colitis ulcerosa.

Die pharmazeutischen Zubereitungen enthalten im allgemeinen zwischen 0,01% bis 10%, vorzugsweise 0,1% bis 1% der erfindungsgemäßen aktiven Komponente(n).

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: Ölige oder alkoholische beziehungsweise wässrige Lösungen sowie Suspensionen und Emulsionen. Bevorzugte Anwendungsformen sind Lösungen, die zwischen 0,01 bis 10 Gewichtsprozent an aktiver Substanz enthalten.

Die Einzeldosis der erfindungsgemäßen aktiven Komponenten kann beispielsweise liegen

a) bei oralen Arzneiformen zwischen 10 - 100 mg/kg, vorzugsweise 20-50 mg/kg.

b) bei parenteralen Arzneiformen (zum Beispiel intravenös, intramuskulär) zwischen 0,1 - 10 mg/kg, vorzugsweise 0,2-0,5 mg/kg.

c) bei Arzneiformen zur Inhalation (Lösungen oder Aerosole) zwischen 1 mg/ml - 30 mg/ml, vorzugsweise 5-15 mg/ml.

- (Die Dosen sind jeweils bezogen auf die freie Base)-

Beispielsweise können 3mal täglich 1 Tablette mit einem Gehalt von 230 bis 2400 mg wirksamer Substanz oder zum Beispiel bei intravenöser Injektion 1mal täglich eine Ampulle von 1 bis 5 ml Inhalt mit 7 bis 70 mg Substanz empfohlen werden. Bei oraler Verabreichung ist die minimale tägliche Dosis beispielsweise 700 mg; die maximale tägliche Dosis bei oraler Verabreichung soll nicht über 7 g liegen.

Die akute Toxizität der erfindungsgemäßen Verbindungen an der Maus (ausgedrückt durch die LD 50 mg/kg; Methode nach Miller und Tainter: Proc. Soc. Exper. Biol. a. Med. 57 (1944) 261) liegt beispielsweise bei oraler Applikation oberhalb 1000 mg/kg.

Nähere Angaben zu den zuvor erwähnten Testmodellen. Die angegebenen Zahlenwerte beziehen sich auf die erfindungsgemäßen Verbindungen, insbesondere die Verbindungen gemäß Beispiel 1.

In-vivo

1. Ovalbumin-induzierte asthmatische Late-Phase Eosinophilie in der Lunge (Meerschweinchen) Lit.: Lung, 169, 1991, S. 227-240

| | |
|---|---|
| 5 mg/kg/i.p.: | 67% Inhibition |
| 10 mg/kg/i.p.: | 98% Inhibition |

2. Haus-Staubmilben-induziertes Asthma (Kaninchen) Lit.: J. Allergy Clin. Immunol. 87, 1991, S. 312

| | |
|---|---|
| Applikation/Dosierung: | 10 mg/ml als Aerosol für 2 Minuten |
| Bronchokonstriktion: | 49%ige Inhibition |
| asthmatische Late-Phase: | 51%ige Inhibition |
| Bronchiale Hyperreaktivität: | 51%ige Inhibition |

3. Allergische Bronchokonstriktion nach Konzett-Rößler (Ratte)

Lit.: Naunyn-Schmiedeb. Arch. Exp. Pathol. Pharmakol. 195, 1940, S. 71-74

0,5 mg/kg i.v.: $ED_{50}$

In vitro

1. Allergisch induzierter Histamine-Release aus Mastzellen (Ratte)

Lit.: J. Immunol. Meth. 30, 1979, S. 55-68

0,7 µmol/l: $IC_{50}$

2. Lipopolysaccharid-induzierte Interleukin1$\beta$Produktion von Monozyten (Mensch)

Lit.: Cell. Immunol. 125, 1990, S. 142-150

4,5 µmol/l: $IC_{50}$

3. Platelet-activating factor (Paf)-induzierte Sauerstoffradikal-Freisetzung aus Alveolarmakrophagen (Meerschweinchen)

Lit.: J. Lipid Med. 5, 1991, S. 13-22

6,1 µmol/l: $IC_{50}$

4. 5-Lipoxygenase-Inhibition in Peritoneal-Makrophagen (Ratte)
12 µmol/l: $IC_{50}$

Die Verbindung mit dem Namen 4,5-Dihydro-8-hydroxy-4-oxo-pyrido [3,2-e]-pyrrolo[1,2-a]pyrazin zeigt bei der Phosphordiesterase-Hemmung eine $IC_{50}$ von 32,2 µmol/l. Die Verbindung gemäß Beispiel 98 zeigt im gleichen Versuch eine $IC_{50}$ von 15,1 µmol/l. Die Verbindung mit der Chiffre D-20169 weist in der Phosphordiesterase-Hemmung einen $IC_{50}$-Wert von 7,34 µmol/l auf.

Beispiele für die Verbindungen der Formel I

Beispiel 1 (Chiffre D 21247)

5-Ethyl-8-[3-(N-cyclohexyl-N-methylcarbamoyl)-propyloxy]-4,5-dihydro-4-oxo-pyrido [3,2-e]-pyrrolo[1,2-a]-pyrazin

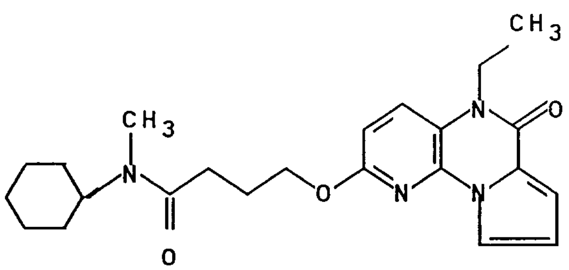

Eine Suspension von 3.8 g (10 mmol) 8-[3-(N-Cyclohexyl-N-methylcarbamoyl)-propyloxy]-4,5-dihydro-4-oxo-pyrido[3,2-e] pyrrolo[1,2-a]Pyrazin in 50 ml Dimethylformamid wird unter Rühren und Schutzgasatmosphäre portionsweise mit 0,26 g (11 mmol) 80-prozentigem Natriumhydrid in Weißöl versetzt. Nach beendeter Zugabe rührt man 2 Stunden bei Raumtemperatur nach und tropft anschließend unter Rühren und Schutzgasatmosphäre (Stickstoff, Argon) eine Lösung von 1,2 g (11 mmol) Ethylbromid in 5 ml Dimethylformamid zu. Nach beendeter Zugabe rührt man 2 Stunden bei 80 °C nach, läßt auf Raumtemperatur abkühlen und filtriert von unlöslichen Bestandteilen ab. Nach Einengen im Vakuum und Umkristallisation aus 2-Propanol erhält man 3,5 g (85 % der Theorie) des angegebenen Reaktionsproduktes als farblose Kristalle.
F. 156-157 °C.

Die Ausgangsverbindung ($R_1$ = H) wird zum Beispiel wie folgt erhalten:
4,0 g (20 mmol)
4,5-Dihydro-8-hydroxy-4-oxo-pyrido [3,2-e]-pyrrolo[1,2-a]pyrazin werden mit 3,9 g (20 mmol) 4-Brombuttersäureethylester umgesetzt. Veretherung der 8-Hydroxy-gruppe analog der Arbeitsweise, die bei der Herstellung von Ausgangssubstanzen beschrieben wird.
Man erhält 1,5 g 4,5-Dihydro-8-[3-ethoxycarbonylpropyloxy]-4-oxo-pyrido[3,2-e]pyrrolo[1,2-a]pyrazin als farblose Kristalle (F. 148-151 °C).

Eine Mischung aus 9,5 g der so erhaltenen 3-Ethoxycarbonyl-Verbindung (30 mmol) 11,2 g (0,2 mmol) Kaliumhydroxid und 250 ml Methanol/Wassergemisch (4:1) wird unter Rühren 16 Stunden unter Rückfluß erhitzt. Nach Abkühlen auf Raumtemperatur säuert man unter Rühren mit konzentrierter Salzsäure an, saugt den ausgefallenen Feststoff ab und wäscht mit Wasser neutral. Anschließend wird das Produkt in Aceton ausgekocht, nach dem Abkühlen erneut abgesaugt und im Vakuum getrocknet. Man erhält 7,9 g 4,5 Dihydro-8-[3-carboxy-propyloxy]-4-oxo-pyrido[3,2-e]pyrrolo [1,2-a]pyrazin als farblose Kristalle (F. 268-272°C).

Zu einer Mischung aus 5,8 g (20 mmol) der so erhaltenen 3-Carboxy-Verbindung, 0,5 ml Dimethylformamid und 300 ml Toluol tropft man unter Rühren und Feuchtigkeitsausschluß 3,2 g (25 mmol) Oxalylchlorid und rührt 2 Stunden bei 50 - 55 °C nach. Anschließend destilliert man das Toluol im Vakuum ab, nimmt den Rückstand in 50 ml Tetrahydrofuran auf und engt erneut im Vakuum bis zur Trockene ein. Der Destillationsrückstand wird in 100 ml Tetrahydrofuran aufgenommen.

Die resultierende Suspension tropft man unter Rühren und Feuchtigkeitsausschluß bei 0-5 °C zu einem Gemisch aus 5,6 g (50 mmol) N-Methylcyclohexylamin und 2,6 g (25 mmol) Natriumcarbonat in 120 ml Tetrahydrofuran/-Wasser-Gemisch (25:1). Nach beendeter Zugabe läßt man unter Rühren auf Raumtemperatur erwärmen und über Nacht nachreagieren. Unlösliche Bestandteile werden abfiltriert, das Filtrat im

Vakuum eingeengt und der Rückstand aus 2-Propanol umkristalltsiert. Man erhält 6,1 g 8-[3-(N-Cyclohexyl-N-methylcarbamoyl)-propyloxy] 4,5-dihydro-4-oxo-pyrido[3,2-e]pyrrolo[1,2-a]pyrazin als farblose Kristalle (F. 192-193 °C).

Analog zu der vorstehend beschriebenen Abhydrolyse einer Alkoxygruppe (Ethoxygruppe) werden folgende Ausgangsverbindungen erhalten:
4,5-Dihydro-8-[4-carboxybutyloxy]-4-oxo-pyrido[3,2-e]-pyrrolo[1,2-a]pyrazin (Chiffre D 20098).
Eine Mischung aus 9,9 g (30 mmol) 4,5-Dihyro-8-[4-ethoxycarbonylbutyloxy]-4-oxo-pyrido[3,2-e]pyrrolo-[1,2-a]pyrazin, 11,2 g (0,2 mol) Kaliumhydroxid und 250 ml Methanol/Wassergemisch (4:1) wird wie vorstehend beschrieben umgesetzt und aufgearbeitet. Nach Trocknen im Vakuum erhält man 8,5 g der Substanz D 20098 als farblose Kristalle, F. 229-233°C.

4,5-Dihydro-8-[carboxy-methoxy]-4-oxo-pyrido[3,2-e]-pyrrolo[1,2-a]pyrazin (Chiffre D 20097). Eine Mischung aus 8,6 g (30 mmol) 4,5-Dihyro-8-[ethoxycarbonylmethoxy]-4-oxo-pyrido[3,2-e]pyrrolo-[1,2-a]-pyrazin, 11,2 g (0,2 mol) Kaliumhydroxid und 250 ml Methanol/Wassergemisch (4:1) wird wie vorstehend beschrieben umgesetzt und aufgearbeitet. Nach Trocknen im Vakuum erhält man 7,3 g der Substanz D 20097 als farblose Kristalle, F. 296-298°C.

Analog der vorstehend beschriebenen Aminierung (Überführung der Carboxylgruppe einer Carboxy-Verbindung in die Chlorcarbonylgruppe und anschließende Umsetzung mit N-Methyl-cyclohexylamin) werden beispielsweise folgende Ausgangsstoffe erhalten:
8-[3-(4-Morpholinylcarbonyl)-propyloxy]-4,5-dihydro-4-oxo-pyrido[3,2-e]-pyrrolo[1,2-a]pyrazin (Chiffre D 19897).
Aus 5,8 g (20 mmol) 4,5-Dihyro-8-[3-carboxypropyloxyl-4-oxo-pyrido[3,2-e]pyrrolo[1,2-alpyrazin und 4,4 g (50 mol) Morpholin erhält man 5,4 g der Substanz D 19897 als farblose Kristalle, F. 205-207°C.

8-[3-(1-Piperidinylcarbonyl)-propyloxy]-4,5-dihydro4-oxo-pyrido[3,2-e]-pyrrolo[1,2-a]pyrazin (Chiffre D 19898).
Aus 5,8 g (20 mmol) 4,5-Dihyro-8-[3-carboxypropyloxy]-4-oxo-pyrido[3,2-e]pyrrolo[1,2-a]pyrazin und 4,3 g (50 mol) Piperidin erhält man 5,5 g der Substanz D 19898 als farblose Kristalle, F. 190-192°C.

8-[3-(N-(4-Fluorbenzyl)-carbamoyl)-propyloxy]-4,5-dihydro-4-oxo-pyrido[3,2-e]-pyrrolo[1,2-a]pyrazin (Chiffre D 19899).
Aus 5,8 g (20 mmol) 4,5-Dihyro-8-[3-carboxypropyloxy]-4-oxo-pyrido[3,2-e]pyrrolo[1,2-a]pyrazin und 6,3 g (50 mol) 4-Fluorbenzylamin erhält man 6,1 g der Substanz D 19899 als farblose Kristalle, F. 246-248°C.

8-[3-(N-Benzylcarbamoyl)-propyloxy]-4,5-dihydro-4-oxo-pyrido[3,2-e]-pyrrolo[1,2-a]pyrazin (Chiffre D 19918).
Aus 5,8 g (20 mmol) 4,5-Dihyro-8-[3-carboxypropyloxy]-4-oxo-pyrido[3,2-e]pyrrolo[1,2-a]pyrazin und 5,6 g (50 mol) Benzylamin erhält man 5,5 g der Substanz D 19918 als farblose Kristalle, F. 232-234°C.

8-[2-(N-Cyclohexyl-N-methylcarbamoyl)-methoxy]-4,5-dihydro-4-oxo-pyrido[3,2-e]-pyrrolo[1,2-a]pyrazin (Chiffre D 20099).
Aus 5,2 g (20 mmol) 4,5-Dihyro-8-[carboxy-methoxy]-4-oxo-pyrido[3,2-e]pyrrolo[1,2-a]pyrazin und 5,6 g (50 mol) N-Methylcyclohexylamin erhält man 5,1 g der Substanz D 20099 als farblose Kristalle, F. 208-209°C.

8-[(4-Morpholinylcarbonyl)-methoxy]-4,5-dihydro-4-oxo-pyrido[3,2-e]-pyrrolo[1,2-a]pyrazin (Chiffre D 20115).
Aus 5,2 g (20 mmol) 4,5-Dihyro-8-[carboxy-methoxy]-4-oxo-pyrido[3,2-e]pyrrolo[1,2-a]pyrazin und 4,4 g (50 mol) Morpholin erhält man 5,0 g der Substanz D 20115 als farblose Kristalle, F. 263-264°C.

8-[(1-Piperidinylcarbonyl)-methoxy]-4,5-dihydro-4-oxo-pyrido[3,2-e]-pyrrolo[1,2-a]pyrazin (Chiffre D 20172).
Aus 5,2 g (20 mmol) 4,5-Dihyro-8-[carboxymethoxyl-4-oxo-pyrido[3,2-e]pyrrolo[1,2-a]pyrazin und 4,3 g (50 mol) Piperidin erhält man 5,0 g der Substanz D 20172 als farblose Kristalle, F. 214-216°C.

8-[(N-Benzylcarbamoyl)-methoxy]-4,5-dihydro-4-oxo-pyrido[3,2-e]-pyrrolo[1,2-a]pyrazin (Chiffre D 20170).
Aus 5,2 g (20 mmol) 4,5-Dihyro-8-[carboxymethoxy]-4-oxo-pyrido[3,2-e]pyrrolo[1,2-alpyrazin und 5,4 g (50 mol) Benzylamin erhält man 5,7 g der Substanz D 20170 als farblose Kristalle, F. 280-281°C.

8-[(N-(4-Fluorbenzyl)-carbamoyl)-methoxy]-4,5-dihydro4-oxo-pyrido[3,2-e]-pyrrolo[1,2-a]pyrazin (Chiffre D 20173).
Aus 5,2 g (20 mmol) 4,5-Dihyro-8-[carboxymethoxy]-4-oxo-pyrido[3,2-e]pyrrolo[1,2-a]pyrazin und 6,3 g (50 mol) 4-Fluorbenzylamin erhält man 5,9 g der Substanz D 20173 als farblose Kristalle, F. 301-304°C.

8-[4-(N-Cyclohexyl-N-methylcarbamoyl)-butyloxyl-4,5-dihydro-4-oxo-pyrido[3,2-e]-pyrrolo[1,2-a]pyrazin (Chiffre D 20169).
Aus 6,0 g (20 mmol) 4,5-Dihyro-8-[4-carboxybutyloxy]-4-oxo-pyrido[3,2-e]pyrrolo[1,2-a]pyrazin und 5,6 g (50

mol) N-Methylcyclohexylamin erhält man 5,7 g der Substanz D 20169 als farblose Kristalle, F. 130-133°C.

8-[4-(N-Benzylcarbamoyl)-butyloxyl-4,5-dihydro-4-oxo-pyrido[3,2-e]-pyrrolo[1,2-a]pyrazin (Chiffre D 20171). Aus 6,0 9 (20 mmol) 4,5-Dihyro-8-[4-carboxybutyloxy]-4-oxo-pyrido[3,2-e]pyrrolo[1,2-a]pyrazin und 5,4 g (50 mol) Benzylamin erhält man 5,9 g der Substanz D 20171 als farblose Kristalle, F. 238-240°C.

8-[4-(4-Morpholinylcarbonyl)-butyloxy]-4,5-dihydro-4-oxo-pyrido[3,2-e]-pyrrolo[1,2-a]pyrazin (Chiffre D 20211).
Aus 6,0 g (20 mmol) 4,5-Dihyro-8-[4-carboxybutyloxy]-4-oxo-pyrido[3,2-e]pyrrolo[1,2-a]pyrazin und 4,4 g (50 mol) Morpholin erhält man 5,5 g der Substanz D 20211 als farblose Kristalle, F. 199-202°C.

8-[4-(1-Piperidinylcarbonyl)-butyloxy]-4,5-dihydro-4-oxo-pyrido[3,2-e]-pyrrolo[1,2-a]pyrazin (Chiffre D 20212).
Aus 6,0 g (20 mmol) 4,5-Dihyro-8-[4-carboxybutyloxy]-4-oxo-pyrido[3,2-e]pyrrolo[1,2-a]pyrazin und 4,3 g (50 mol) Piperidin erhalt man 5,5 g der Substanz D 20212 als farblose Kristalle, F. 191-193°C.

Beispiel 2a (Chiffre D 19592)

1-Chlor-5-hydroxy-4,5-dihydro-4-oxo-pyrido[3,2-e] pyrrolo[1,2-a]pyrazin

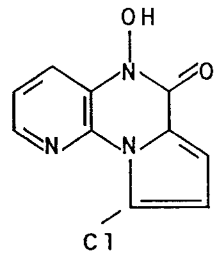

Eine Lösung aus 50 g (178 mmol) 2-[1-(2-Ethoxycarbonyl-4-hydroxy)-pyrrolidinyl]-3-nitro-pyridin (hergestellt aus 2-Chlor-3-nitro-pyridin und Hydroxyprolinethylester; siehe Herstellung von Ausgangsstoffen) und 38 g Ammoniumchlorid (0,7 mmol in 1200 ml Ethanol/Wasser-Gemisch (1:1) wird unter Rühren portionsweise mit 46,5 g Zinkstaub versetzt. Dabei steigt die Temperatur allmählich auf 40 °C an. Nach beendeter Zugabe rührt man 1 Stunde bei 40 - 45 °C nach. Nach dem Abkühlen auf Raumtemperatur filtriert man ab und extrahiert den zurückbleibenden Feststoff viermal mit je 200 ml Dimethylacetamid. Die vereinigten Extrakte werden im Vakuum eingeengt und der verbleibende Rückstand unter Rühren in ethanolische Salzsäure eingetragen. Nach Stehen über Nacht bei 0-4 °C saugt man ab, nimmt den Feststoff in 1,5 Liter heißem Methanol auf und rührt 20 Tage unter Durchleiten von Luft bei Raumtemperatur nach. Der kristalline Niederschlag wird abgesaugt und mit Methanol nachgewaschen. Nach Umkristallisation aus Dioxan erhält man 11,5 g (27 % der Theorie) des angegebenen Reaktionsproduktes als beigefarbene Kristalle (F. 265-267 °C.).

Beispiel 2b (Chiffre D 19529)

Formel wie Beispiel 2a, jedoch ohne das Chlor in 1-Stellung.
Herstellung: analog Beispiel 2a
F.: 235 - 236°C

Beispiel 3 (Chiffre D 19696)

1-Chlor-5-benzyloxy-4,5-dihydro-4-oxo-pyrido[3,2-e] pyrrolo[1,2-a]pyrazin

Eine Suspension von 2,4 g (10 mmol 1-Chlor-5-hydroxy-4,5-dihydro-4-oxo-pyrido[3,2-e]pyrrolo[1,2-alpyrazin (Beispiel 2) in 50 ml Dimethylformamid wird unter Rühren und Schutzgasatmosphäre portionsweise mit 0,26 g (11 mmol 80-prozentigem Natriumhydrid in Weißöl versetzt. Nach beendeter Zugabe rührt man 2 Stunden bei Raumtemperatur nach und tropft anschließend unter Rühren und Schutzgasatmosphäre eine Lösung von 2,0 g (11 mmol) Benzylbromid in 5 ml Dimethylformamid zu. Nach beendeter Zugabe rührt man 2 Stunden bei 80 °C nach, läßt auf Raumemperatur abkühlen und filtriert daraufhin von unlöslichen Bestandteilen ab. Nach Einengen im Vakuum und Umkristallisation aus Aceton erhält man 1,9

(58 % der Theorie) des angegebenen Reaktionsproduktes als farblose Kristalle.
F. 179-180 °C.

Beispiel 4a (Chiffre D 19668)

1-Chlor-5-(N,N-diethylcarbamoyloxy)-4,5-dihydro-4-oxo-pyrido[3, 2-e] pyrrolo[1,2-a]pyrazin

Eine Suspension von 2,4 g (10 mmol) 1-Chlor-5-hydroxy-4,5-dihydro-4-oxo-pyrido[3,2-e]pyrrolo [1,2-a]-pyrazin (Beispiel 2) in 50 ml Dimethylformamid wird analog Beispiel 3 mit 1,5 9 (11 mmol) N,N-Diethylcarbamoylchlorid umgesetzt. Nach Umkristallisation aus Essigester erhält man 1,1 g (33 % der Theorie) des angegebenen Reaktionsproduktes als farblose Kristalle. F. 173-175 °C.

Beispiel 4b (Chiffre D 19537)

5-Morpholinocarbonyloxy-4,5-dihydro-4-oxo-pyrido-[3,2-e]pyrrolo[1,2-a]pyrazin

F. 176-177°C.

Beispiel 4c (Chiffre D 19591)

1-Chlor-5-Ethyloxycarbonyloxy-4,5-dihydro-4-oxo-pyrido-[3,2-e]pyrrolo[1,2-a]pyrazin

F. 163-165°C

Beispiel 4d (Chiffre D 19621)

1-Chlor-5-dimethylaminocarbonyloxy-4,5-dihydro-4-oxo-pyrido-[3,2-e]pyrrolo[1,2-a]pyrazin

F. 210-211°C

Beispiel 4e (Chiffre D 19629)

1-Chlor-5-morpholinocarbonyloxy-4,5-dihydro-4-oxo-pyrido-[3,2-e]pyrrolo[1,2-a]pyrazin

F. 204-205°C

Beispiel 4f (Chiffre D 19669)

1-Chlor-5-acetoxy-4,5-dihydro-4-oxo-pyrido-[3,2-e]pyrrolo[1,2-a]pyrazin

F. 196-198°C

Beispiel 4g (Chiffre D 19700)

1-Chlor-5-propen(2)-oxy-4,5-dihydro-4-oxo-pyrido-[3,2-e]pyrrolo[1,2-a]pyrazin

F. 104-106°C

Beispiel 4h (Chiffre D 19711)

1-Chlor-5-propin-(2)-oxy-4,5-dihydro-4-oxo-pyrido-[3,2-e]pyrrolo[1,2-alpyrazin

F. 205-207°C
Die Herstellung der Verbindungen gemäß den Beispielen 4b bis 4f erfolgt analog Beispiel 4a.

Beispiele 5 bis 10

Beispiele für Verbindungen der Formel I worin $R_1$ $C_1$-$C_6$-Halogenalkyl ist.

Eine Suspension von 0,5 mol einer Verbindung der Formel I, worin $R_1$ Wasserstoff ist, in 1000 ml Dimethylformamid (oder Dimetylacetamid oder N-Methylpyrrolidon) wird unter Rühren und Feuchtigkeitsausschluß portionsweise mit 16,5 bis 22,5 g (0,55-0,75 mol) 80-prozentigem Natriumhydrid in Weißöl versetzt. Nach beendeter Zugabe rührt man 2 Stunden bei Raumtemperatur nach und tropft das Reaktionsgemisch anschließend unter Rühren und Schutzgasatmosphäre zu einer Lösung von 1-10 mol des entsprechenden 1-Brom-ω-chloralkans bzw. 1,ω-Dihalogenalkans worin beide Halogenatome gleich sind in Dimethylformamid (oder Dimethylacetamid oder N-Methylpyrrolidon). Nach beendeter Zugabe rührt man 3 Stunden bei Raumtemperatur nach und filtriert daraufhin von unlöslichen Bestandteilen ab. Nach Einengen im Vakuum und Umkristallisation aus Acetonnitril erhält man je nach eingesetztem 1,ω-Dihalogenalkan die reinen ω-Halogenalkylderivate oder Gemische der korrespondierenden ω-Brom-beziehungsweise ω-Chloralkyl-Verbindungen, deren Verhältnis aus 1H-NMR-Daten ermittelt werden kann. Für weitere Umsetzungen können diese Halogenalkyl-Verbindungen beziehunsweise die entsprechenden Gemische ohne weitere Aufarbeitung eingesetzt werden.

Beispiel 5 (Chiffre RD 680/Bi6066)

4:1-Gemisch aus 5-(2-Chlorethyl)- und 5-(2-Bromethyl)-4,5-dihydro-8-methoxy-4-oxo-pyrido[3,2-e]-pyrrolo [1,2-a]pyrazin:

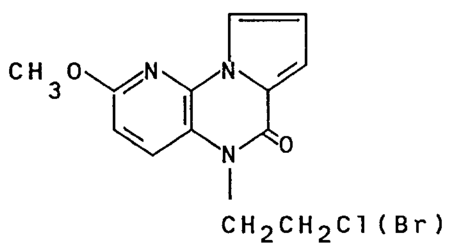

Farblose bis beige Kristalle. Das Gemisch schmilzt bei 172-173 °C.
Ausbeute: 52 % der Theorie.

Beispiel 6 (Chiffre RD 537/Bi6017)

3:2-Gemisch aus 5-(3-Brompropyl)- und 5-(3-Chlorpropyl)-4,5-dihydro-8-methoxy-4-oxo-pyrido [3,2-e]-pyrrolo[1,2-alpyrazin.
Farblose bis beige Kristalle. F.105-107 °C.
Ausbeute 68 % der Theorie.

Beispiel 7 (Chiffre RD 541/UE 3)

9:1-Gemisch aus 5-(3-Chlorpropyl)- und 5-(3-Brompropyl)4,5-dihydro-4-oxo-pyrido[3,2-e]pyrrolo [1,2-a]-pyrazin
Farblose bis beige Kristalle. F. 144-145 °C.
Ausbeute: 72 % der Theorie

Beispiel 8 (Chiffre RD 781/MB8)

5-(6-Bromhexyl)-4,5-Dihydro-4-oxo-pyrrolo [1,2-a]chinoxalin

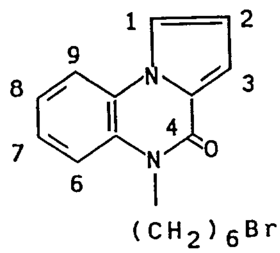

Farblose bis beige Kristalle. F. 83-85 °C.
Ausbeute: 77 % der Theorie

Beispiel 9 (Chiffre RD 789/T7627)

3:2-Gemisch aus 5-(4-Brombutyl- und 5-(4-Chlorbutyl)--4,5-dihydro-8-methyl-4-oxo-pyrrolo [1,2-a]-chinoxalin
Farblose bis beige Kristalle. F. 87-88 °C.
Ausbeute: 49 % der Theorie

Beispiel 10 (Chiffre RD 790/T7632).

7:3-Gemisch aus 5-(3-chlor-2-methylpropyl)- und 5-(3-brom-2-methylpropyl)-4,5-dihydro-4-oxo-pyrrolo [1,2-a]chinoxalin
Farblose bis beige Kristalle. F.122-124 °C.
Ausbeute: 53 % der Theorie

Beispiele 11 - 97

Die Verbindungen dieser Beispiele werden durch die folgende allgemeine Formel und die zugehörige Tabelle 1 dargestellt.

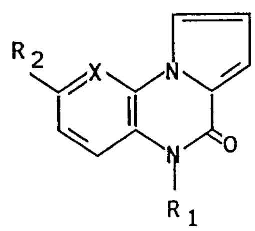

Tabelle 1

| Beispiel Nr. | X | $R_1$ | $R_2$ | F °C | Chiffre |
|---|---|---|---|---|---|
| 11 | N | 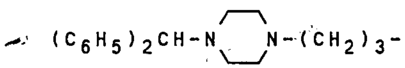 | H | 225-228 Dihydrochlorid | D 20896 |
| 12 | N | 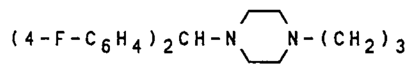 | H | 173-175 | D 20897 |
| 13 | N | 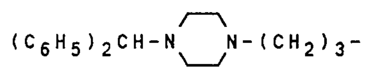 | 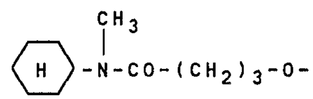 | 192-195 Dihydrochlorid | D 21280 |
| 14 | N | 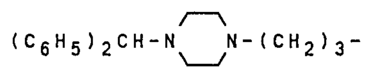 | " | 178-182 Dihydrochlorid | D 21524 |
| 15 | N | 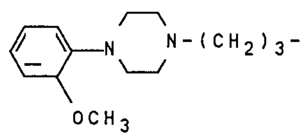 | " | 184-188 Hydrochlorid | D 21525 |
| 16 | N | 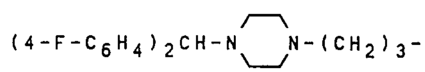 | " | 200 Dihydrochlorid | D 21616 |

Tabelle 1

| Beispiel Nr. | X | $R_1$ | $R_2$ | F °C | Chiffre |
|---|---|---|---|---|---|
| 17 | N | 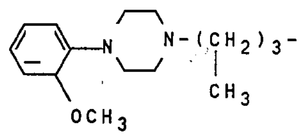 | H | 204-209 Hydrochlorid | D 21617 |
| 18 | CH | 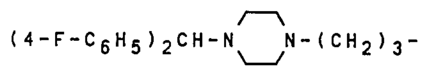 | H | 220-224 Dihydrochlorid | D 21157 |
| 19 | N | 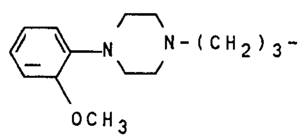 | H | 213-216 Dihydrochlorid | D 21010 |
| 20 | CH | 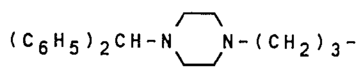 | H | 228-230 Dihydrochlorid | D 21400 |
| 21 | CH | 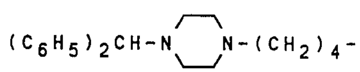 | H | 243-250 Dihydrochlorid | D 21402 |

Tabelle 1

| Beispiel Nr. | X | $R_1$ | $R_2$ | F °C | Chiffre |
|---|---|---|---|---|---|
| 22 | CH | 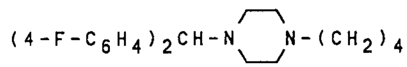 | H | 165-167 | D 21401 |
| 23 | CH | 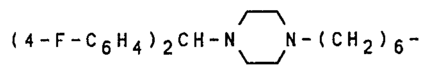 | H | 206-212 Dihydrochlorid | D 21619 |
| 24 | CH | 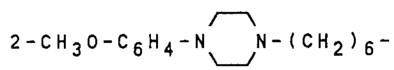 | H | 172-179 Dihydrochlorid | D 21618 |
| 25 | CH | 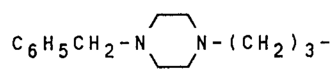 | H | 229-232 Dihydrochlorid | D 21117 |
| 26 | N | 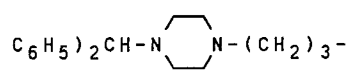 | $CH_3O$ | 220-226 Dihydrochlorid 1 Mol $H_2O$ | D 20971 |
| 27 | N | 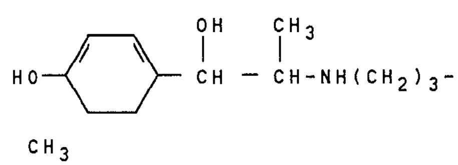 | $CH_3O$ | 218-219 Hydrochlorid | D 21237 |

Tabelle 1

| Beispiel Nr. | X | $R_1$ | R2 | F °C | Chiffre |
|---|---|---|---|---|---|
| 28 | N | 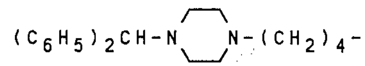 | $CH_3O$ | 175 | D 21271 |
| 29 | N | 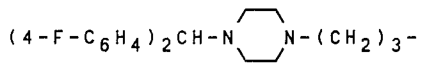 | $CH_3O$ | 216-219<br>Dihydrochlorid<br>1 Mol $H_2O$ | D 20972 |
| 30 | N | 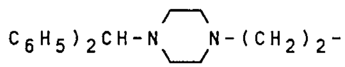 | $CH_3O$ | 180<br>Dihydrochlorid | D 21206 |
| 31 | N | 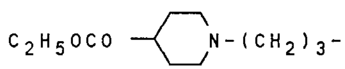<br>HBr | $CH_3O$ | 180<br>Hydrobromid | D 20898 |
| 32 | N | 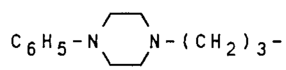 | $CH_3O$ | 224-226<br>Dihydrochlorid<br>1 Mol $H_2O$ | D 20944 |
| 33 | N | 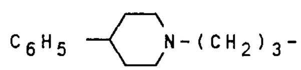 | $CH_3O$ | 241-245<br>Hydrochlorid<br>1 Mol $H_2O$ | D 20973 |

Tabelle 1

| Beispiel Nr. | X | $R_1$ | $R_2$ | F °C | Chiffre |
|---|---|---|---|---|---|
| 34 | N | $4-F-C_6H_5-CH_2-NH-(CH_2)_3-$ HBr | $CH_3O$ | 206 Hydrobromid | D 20974 |
| 35 | N | 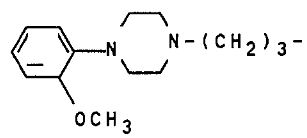 | $CH_3O$ | 143-146 Dihydrochlorid | D 21008 |
| 36 | N | 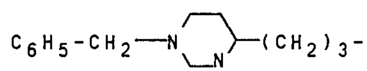 HBr | $CH_3O$ | 188-191 Hydrobromid | D 21009 |
| 37 | N | $C_6H_5-(CH_2)_3-NH(CH_2)_3-$ | $CH_3O$ | 173-174 Hydrochlorid | D 21045 |
| 38 | N | $C_6H_5-CH_2-NH(CH_2)_3-$ | $CH_3O$ | 221-223 Hydrochlorid | D 21046 |
| 39 | N | 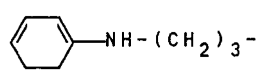 | $CH_3O$ | 233-235 Hydrochlorid | D 21081 |

Tabelle 1

| Beispiel Nr. | X | $R_1$ | R2 | F °C | Chiffre |
|---|---|---|---|---|---|
| 40 | N | 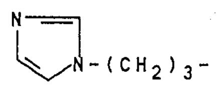 | $CH_3O$ | 197-199 Hydrochlorid | D 21088 |
| 41 | N | 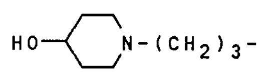 | $CH_3O$ | 103-105 | D 21089 |
| 42 | N | 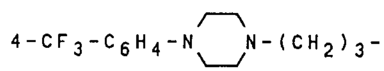 | $CH_3O$ | 203-205 | D 21090 |
| 43 | N | 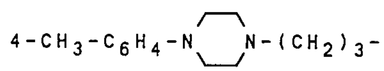 | $CH_3O$ | 229-231 Hydrochlorid | D 21159 |
| 44 | N | 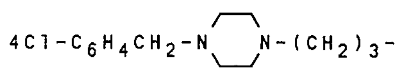 | $CH_3O$ | 245-248 Dihydrochlorid | D 21205 |
| 45 | N | 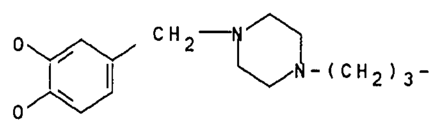 | $CH_3O$ | 225-228 Dihydrochlorid | D 21207 |

Tabelle 1

| Beispiel Nr. | X | $R_1$ | R2 | F °C | Chiffre |
|---|---|---|---|---|---|
| 46 | N | 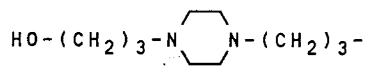 | $CH_3O$ | 226-228 Dihydrochlorid | D 21236 |
| 47 | N | 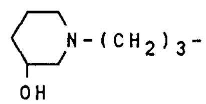 | $CH_3O$ | 225-228 Hydrochlorid | D 21272 |
| 48 | N | 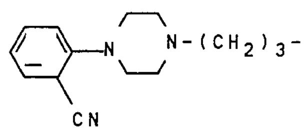 | $CH_3O$ | 224-226 Hydrochlorid | D 21273 |
| 49 | N | 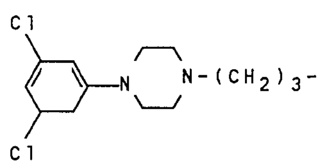 | $CH_3O$ | 254-256 Hydrochlorid | D 21387 |
| 50 | N | $H_2N-(CH_2)_3-$ | $CH_3O$ | 261-263 Hydrochlorid | D 21158 |

Tabelle 1

| Beispiel Nr. | X | $R_1$ | $R_2$ | F °C | Chiffre |
|---|---|---|---|---|---|
| 51 | N | 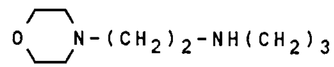 | $CH_3O$ | 215-220 Dihydrochlorid | D 21161 |
| 52 | N | 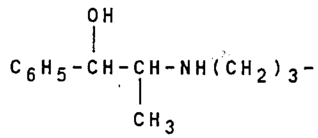 | $CH_3O$ | 223-225 Hydrochlorid L-Erythro-Form | D 21160 |
| 53 | N | 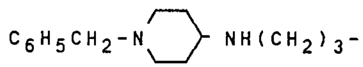 | $CH_3O$ | 259-261 Dihydrochlorid | D 21162 |
| 54 | N | 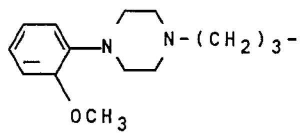 | Cl | 228 Hydrochlorid | D 21155 |
| 55 | N | 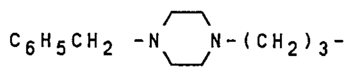 | Cl | 204-208 Dihydrochlorid | D 21156 |
| 56 | N | 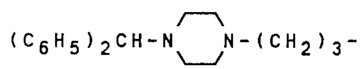 | Cl | 212-217 Dihydrochlorid 1 Mol Wasser | D 21526 |
| 57 | N | 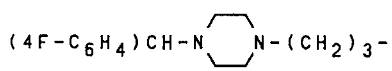 | Cl | 208-212 Dihydrochlorid | D 21527 |

Tabelle 1

| Beispiel Nr. | X | $R_1$ | $R_2$ | F °C | Chiffre |
|---|---|---|---|---|---|
| 58 | N | $C_6H_5CH_2$- | $CH_3O$ | 126-127 | D 18540 |
| 59 | N | $C_6H_5(CH_2)_2$- | $CH_3O$ | 112-114 | D 18541 |
| 60 | N | $C_6H_5(CH_2)_3$- | $CH_3O$ | 98-99 | D 18542 |
| 61 | N | $CH_3CH_2$- | $CH_3O$ | 106-112 | D 18688 |
| 62 | N | $CH_3(CH_2)_2$- | $CH_3O$ | 88-89 | D 18689 |
| 63 | N | $CH_3(CH_2)_3$- | $CH_3O$ | 83-86 | D 18690 |
| 64 | N | $C_6H_5CH_2$- | H | 138-141 | D 18697 |
| 65 | N | $CH_3$- | $CH_3O$ | > 160 (Zersetzung) | D 18999 |
| 66 | N | $C_6H_5CH_2$- | H | > 176 (Zersetzung) | D 19000 |
| 67 | N | $C_6H_5(CH_2)_2$- | H | > 131 (Zersetzung) | D 19001 |
| 68 | CH | $C_6H_5(CH_2)_3$- | H | > 85 (Zersetzung) | D 19002 |
| 69 | CH | $C_6H_5CO$- | H | > 180 (Zersetzung) | D 19003 |
| 70 | N | $C_6H_5CO$- | H | > 179 (Zersetzung) | D 19004 |
| 71 | N | $(CH_3)_2N-(CH_2)_2$- | $CH_3O$ | 246-247 Hydrochlorid | D 19038 |

Tabelle 1

| Beispiel Nr. | X | $R_1$ | $R_2$ | F °C | Chiffre |
|---|---|---|---|---|---|
| 72 | N | $(CH_3)_2N-(CH_2)_2-$ | H | 292-293 Hydrochlorid | D 19126 |
| 73 | CH | $(CH_3)_2N-(CH_2)_2-$ | H | 285-287 Hydrochlorid | D 19127 |
| 74 | N | $(CH_3)_2N-(CH_2)_3-$ | $CH_3O$ | 229-231 Hydrochlorid | D 19128 |
| 75 | N | $(CH_3)_2N-(CH_2)_3-$ | H | 276-278 Hydrochlorid | D 19129 |
| 76 | CH | $(CH_3)_2N-(CH_2)_3-$ | H | 208-211 Hydrochlorid | D 19130 |
| 77 | N | $(C_2H_5)_2N(CH_2)_2-$ | $CH_3O$ | 217-219 Hydrochlorid | D 19131 |
| 78 | N | $(C_2H_5)_2N(CH_2)_2-$ | H | 208-211 Hydrochlorid | D 19132 |
| 79 | CH | $(C_2H_5)_2N(CH_2)_2-$ | H | 202-205 Hydrochlorid | D 19133 |
| 80 | N | $(C_2H_5)_2N(CH_2)_3-$ | H | 210-220 Hydrochlorid | D 19134 |
| 81 | CH | $(C_2H_5)_2N(CH_2)_3-$ | H | 175-177 Hydrochlorid | D 19135 |

Tabelle 1

| Beispiel Nr. | X | $R_1$ | $R_2$ | F °C | Chiffre |
|---|---|---|---|---|---|
| 82 | N | 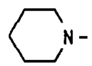 $N-(CH_2)_3-$ | H | 218-220 Hydrochlorid | D 19136 |
| 83 | CH | 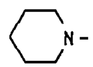 $N-(CH_2)_3-$ | H | 188-191 Hydrochlorid | D 19137 |
| 84 | N | $(C_2H_5)_2N-(CH_2)_3$ | $CH_3O$ | 217-218 Hydrochlorid | D 19218 |
| 85 | CH | 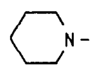 $N-(CH_2)_2-$ | H | 260-263 Hydrochlorid | D 19219 |
| 86 | N | $(CH_3)_2N-CH_2-\underset{CH_3}{\underset{\mid}{CH}}-$ | $CH_3O$ | 217-218 Hydrochlorid | D 19221 |
| 87 | N | $(CH_3)_2N-CH_2-\underset{CH_3}{\underset{\mid}{CH}}-$ | H | 284-290 Hydrochlorid | D 19223 |
| 88 | N | 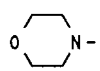 $N-(CH_2)_2-$ | H | 257-258 Hydrochlorid | D 19224 |

Tabelle 1

| Beispiel Nr. | X | $R_1$ | $R_2$ | F °C | Chiffre |
|---|---|---|---|---|---|
| 89 | N | 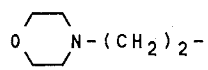 | $CH_3O$ | 213-216 Hydrochlorid | D 19225 |
| 90 | N | 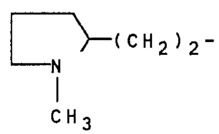 | H | 232-233 Hydrochlorid | D 19226 |
| 91 | CH | 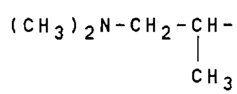 | H | 268-270 Hydrochlorid | D 19324 |
| 92 | CH | 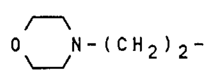 | H | 227-229 Hydrochlorid | D 19325 |
| 93 | N | 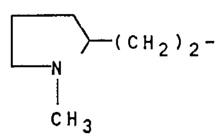 | H | 181-184 Hydrochlorid | D 19326 |

Tabelle 1

| Beispiel Nr. | X | $R_1$ | $R_2$ | F °C | Chiffre |
|---|---|---|---|---|---|
| 94 | N | $CH_3-CH(OH)-CH_2-$ | H | 181-184 | D 19327 |
| 95 | N | $CH_2(OH)-CH(OH)-CH_2-$ | $CH_3O$ | 160-164 | D 19328 |
| 96 | N | $CH_2(OH)-CH(OH)-CH_2-$ | H | 164-166 | D 19456 |
| 97 a | N | $CH\equiv C-CH_2$ | $CH\equiv C-CH_2O$ | 218-220 | D 19800 |
| 98 | N | H | 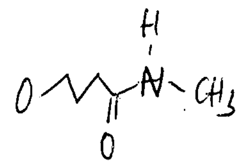 | 262-264 | D 20239 |
| 99 | N | H | 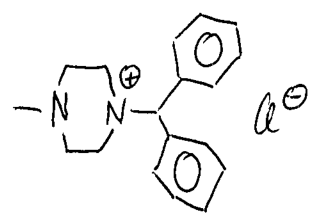 | > 250 | D 20466 |

Allgemeine Vorschrift zur Herstellung der Verbindungen der Tabelle 1

Eine Lösung aus 2,7 g (10 mmol) eines 9:1-Gemisches aus 5-(3-Chlopropyl)- und 5-(3-Brompropyl)-4,5-dihydro-4-oxo-pyrido [3,2-e]pyrrolo[1,2-a]pyrazin (Beispiel 7) 2,6 g (10,5 mmol) 1-Diphenylmethylpiperazin und 1,1 g (11 mmol) Triethylamin in 50 ml Dimethylformamid wird unter Rühren und Schutzgasatmosphäre

3 Stunden auf 120-130 °C erhitzt. Nach Abkühlen auf Raumtemperatur wird das Reaktionsgemisch bis zur Trockene eingeengt und der Rückstand in 150 ml Dichlormethan aufgenommen. Die Dichlormethanphase wird je einmal mit 30 ml 2N Salzsäure und 30 ml 2N Natronlauge extrahiert und anschließend dreimal mit je 40 ml Wasser neutral gewaschen. Nach Trocknen mit Natriumsulfat engt man die Dichlormethanphase im Vakuum ein, nimmt den Rückstand in 80 ml Ethanol auf und bringt durch Zugabe von ethanolischer Salzsäure zur Kristallisation. Das erhaltene Kristallisat wird abgesaugt, mit Methanol nachgewaschen und im Vakuum getrocknet.

Ausgangskomponenten für die Beispiele 13, 14 und 15 sind jeweils ein 2:1-Gemisch aus 5-(3-Brompropyl)- und 5-(3-Chlorpropyl)-8-[3-(N-Cyclohexyl-N-methylcarbamoyl)-propylo xy]-4,5-dihydro-4-oxo-pyrido[3,2-e]pyrrolo[1,2-a]pyrazin sowie 1 Benzyl-piperazin (Beispiel 13), 1-Diphenylmethylpiperazin (Beispiel 14) und 1(2-Methoxy-phenyl-piperazin) (Beispiel 15). Bei Beispiel 17 sind die Ausgangskomponenten ein 1:1-Gemisch aus 5-(3-Brompropyl)- und 5-(3-Chlorpropyl)-8-[3-(N-Cyclohexyl-N-methylcarbamoyl )-propylo xy]-4,5-dihydro-4-oxo-pyrido[3,2-e]pyrrolo[-1,2a]pyrazin (Beispiel 10) und 1-(2-Methoxy-phenyl)-piperazin.

Für die Beispiele 19 und 20 ist die eine Ausgangskomponente jeweils ein 4:1-Gemisch aus 5-(3-Chlorpropyl)- und 5-(3-Brompropyl)-4,5-dihydro-4-oxo-pyrrolo[1,2-a]chinoxalin und die andere 1-(2-Methoxy-phenyl)-piperazin (Beispiel 19) und 1-Diphenylmethylpiperazin (Beispiel 20).
Für die Beispiele 21 und 22 ist die eine Ausgangskomponente jeweils ein 3:2-Gemisch aus 5-(6-Bromhexyl)-4,5-dihydro-4-oxo-pyrrolo[1,2-a]-chinoxalin (siehe Beispiel 9) und die andere 1-Diphenyl-methyl-piperazin (Beispiel 21) beziehungsweise 1-(Di-(4-fluor-phenyl)-methyl-piperazin.
Für die Beispiele 23 und 24 ist die eine Ausgangskomponente jeweils 5-(6-Bromhexyl)-4,5-dihydro-4-oxo-pyrrolo[1,2-a]-chinoxalin (siehe Beispiel 8) und die andere 1-(Di-C4-fluor-phenyl)-methyl)piperazin (Beispiel 23) beziehungsweise 1-(2-Methoxy-phenyl)-piperazin (Beispiel 24).

Selbstverständlich können auch andere 5-Halogenalkyl-Gemische als die oben angegebenen beziehungsweise auch die reinen 5-Halogenalkylverbindungen eingesetzt werden.

**Patentansprüche**

1. Verbindungen der Formel

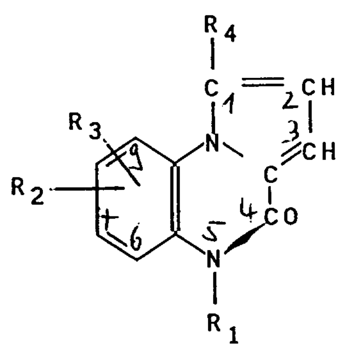

I

worin der Phenylring entweder in Stellung 6, 7, 8 oder 9 anstelle einer CH-Gruppe auch ein Stickstoffatom enthalten kann und die Reste $R_1$, $R_2$, $R_3$ und $R_4$ die folgenden Bedeutungen haben:

$R_1$: $C_2$-$C_6$-Alkenyl, $C_2$-$C_6$-Alkinyl, Hydroxy, $C_1$-$C_6$-Alkoxy, $C_3$-$C_6$-Alkenyloxy, $C_3$-$C_6$-Alkinyloxy, $C_2$-$C_6$-Alkanoyloxy, Benzoyloxy, Morpholinocarbonyloxy, $C_1$-$C_6$-Alkyloxycarbonyloxy, $C_1$-$C_6$-Alkylaminocarbonyloxy, $C_1$-$C_6$-Dialkylaminocarbonyloxy oder die Gruppe

-Alk-A

worin Alk: $C_1$-$C_6$-Alkyl, $C_2$-$C_6$-Hydroxy-alkyl oder $C_3$-$C_6$-Cycloalkyl ist und das Symbol A bedeutet:

1. Wasserstoff, Halogen, Hydroxy, $C_1$-$C_6$-Alkoxy, $C_2$-$C_6$-Alkanoyloxy, Phenyl;
2. $-NHR_5$, $-NR_5R_6$, $NR_5R_6R_7$, Pyridylamino, Imidazolyl, Pyrrolidinyl, N-$C_1$-$C_6$-Alkylpyrrolidinyl, Piperidylamino, N-(Phenyl-$C_1$-$C_4$-alkyl)-piperidylamino, wobei $R_5$ und $R_6$ gleich oder verschieden sind und Wasserstoff, $C_1$-$C_6$-Alkyl, $C_3$-$C_7$-Cycloalkyl, $C_3$-$C_7$-Hydroxycyloalkyl, Morpholino-$C_1$-$C_6$-Alkyl, Phenyl, Phenyl-$C_1$-$C_6$-alkyl oder Phenyl-$C_2$-$C_6$-oxyalkyl dar-

stellen, wobei die Phenylreste auch durch Halogen substituiert sein können und $R_7$ Wasserstoff oder $C_1$-$C_6$-Alyl ist;
3. Die Gruppe

-CO-D

worin D Phenyl, $C_1$-$C_6$-Alkyl, $C_3$-$C_7$-Cycloalkyl, Hydroxy, $C_1$-$C_6$-Alkoxy, $C_3$-$C_7$-Cycloalkyloxy, Morpholino, Pyrrolidino, Piperidino, Homopiperidino, Piperazino, -$NHR_5$ oder -$NR_5R_6$ ist und $R_5$ und $R_6$ die angegebenen Bedeutungen haben.
4. Die Gruppe

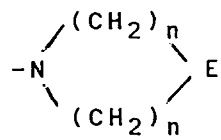

worin n die Zahlen 1-3 annehmen kann und E $CH_2$, Sauerstoff, Schwefel, NH, CHOH, CH-$C_1$-$C_6$-Alkyloxy, CH-$C_2$-$C_6$-Alkanoyloxy, $CHC_6H_5$, CHCOD, CH-$CN_2C_6H_5$, N-$C_1$-$C_6$-Alkyl, N-$C_1$-$C_6$-Hydroxyalkyl, N-$C_6H_5$, N-$CH_2C_6H_5$, N-$CH(C_6H_5)_2$, N-$(CH_2)_2$-OH, N-$(CH_2)_3$-OH oder NCOD darstellt und die Phenylreste ($C_6H_5$) auch durch Halogen, $C_1$-$C_6$-Alkoxy, Trifluormethyl, $C_1$-$C_6$-Alkyl, Methylendioxy, Cyan substituiert sein können und D die oben angegebenen Bedeutungen hat;

$R_2$ und $R_3$, die gleich oder verschieden sein können:
Wasserstoff, Halogen, Hydroxy, $C_1$-$C_6$-Alkyl, Trifluormethyl, -CN, $C_1$-$C_6$-Alkoxy, $C_3$-$C_6$-Alkenyloxy, $C_3$-$C_6$-Alkinyloxy, -$NHR_5$, -$NR_5R_6$, $NR_5R_6R_7$ (Bedeutungen $R_5$, $R_6$, $R_7$ wie angegeben), oder die Gruppe -G-Alk-A, wobei Alk und A die angegebenen Bedeutungen haben und G Sauerstoff, Schwefel, NH oder $NR_5$ ist;
$R_4$: Wasserstoff oder Halogen,
wobei $R_1$ auch Wasserstoff sein kann, wenn $R_2$ die Gruppe

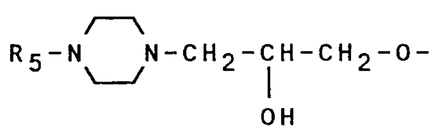

ist und $R_5$ Phenyl, $C_1$-$C_4$-Alkoxy-phenyl oder Diphenylmethyl darstellen und $R_3$ und $R_4$ Wasserstoff sind,
und deren physiologisch verträglichen Säureadditionssalze und quartären Ammoniumsalze, ausgenommen die Verbindungen der Formel I, wo $R_1$ Methyl, Dimethylamino-propyl, Dimethylamino-ethyl, Morpholino-ethyl oder Pyrrolidino-ethyl ist, $R_2$, $R_3$ und $R_4$ Wasserstoff sind und der Phenylring kein Stickstoffatom enthält.

**2.** Verfahren zur Herstellung von Verbindungen der Formel

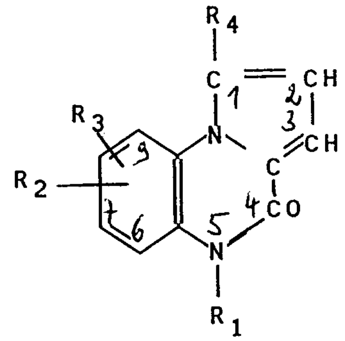

I

worin der Phenylring entweder in Stellung 6, 7, 8 oder 9 anstelle einer CH-Gruppe auch ein Stickstoffatom enthalten kann und die Reste $R_1$, $R_2$, $R_3$ und $R_4$ die folgenden Bedeutungen haben:

$R_1$: $C_2$-$C_6$-Alkenyl, $C_2$-$C_6$-Alkinyl, Hydroxy, $C_1$-$C_6$-Alkoxy, $C_3$-$C_6$-Alkenyloxy, $C_3$-$C_6$-Alkinyloxy, $C_2$-$C_6$-Alkanoyloxy, Benzoyloxy, Morpholinocarbonyloxy, $C_1$-$C_6$-Alkyloxycarbonyloxy, $C_1$-$C_6$-Alkylaminocarbonyloxy, $C_1$-$C_6$-Dialkylaminocarbonyloxy oder die Gruppe

-Alk-A

worin Alk: $C_1$-$C_6$-Alkyl, $C_2$-$C_6$-Hydroxy-alkyl oder $C_3$-$C_6$-Cycloalkyl ist und das Symbol A bedeutet:

1. Wasserstoff, Halogen, Hydroxy, $C_1$-$C_6$-Alkoxy, $C_2$-$C_6$-Alkanoyloxy, Phenyl;
2. $-NHR_5$, $-NR_5R_6$, $NR_5R_6R_7$, Pyridylamino, Imiuazolyl, Pyrrolidinyl, N-$C_1$-$C_6$-Alkylpyrrolidinyl, Piperidylamino, N-(Phenyl-$C_1$-$C_4$-alkyl)-piperidylamino, wobei $R_5$ und $R_6$ gleich oder verschieden sind und Wasserstoff, $C_1$-$C_6$-Alkyl, $C_3$-$C_7$-Cycloalkyl, $C_3$-$C_7$-Hydroxycyloalkyl, Morpholino-$C_1$-$C_6$-Alkyl, Phenyl, Phenyl-$C_1$-$C_6$-alkyl oder Phenyl-$C_2$-$C_6$-oxyalkyl darstellen, wobei die Phenylreste auch durch Halogen substituiert sein können und $R_7$ Wasserstoff oder $C_1$-$C_6$-Alkyl ist;
3. Die Gruppe

-CO-D

worin D Phenyl, $C_1$-$C_6$-Alkyl, $C_3$-$C_7$-Cycloalkyl, Hydroxy, $C_1$-$C_6$-Alkoxy, $C_3$-$C_7$-Cycloalkyloxy, Morpholino, Pyrrolidino, Piperidino, Homopiperidino, Piperazino, $-NHR_5$ oder $-NR_5R_6$ ist und $R_5$ und $R_6$ die angegebenen Bedeutungen haben.

4. Die Gruppe

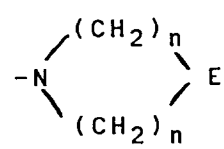

worin n die Zahlen 1-3 annehmen kann und E $CH_2$, Sauerstoff, Schwefel , NH, CHOH, CH-$C_1$-$C_6$-Alkyloxy, CH-$C_2$-$C_6$-Alkanoyloxy, $CHC_6H_5$, CHCOD, $CH$-$CH_2C_6H_5$, N-$C_1$-$C_6$-Alkyl, N-$C_1$-$C_6$-Hydroxyalkyl, N-$C_6H_5$, N-$CH_2C_6H_5$, N-$CH(C_6H_5)_2$, N-$(CH_2)_2$-OH, N-$(CH_2)_3$-OH oder NCOD darstellt und die Phenylreste ($C_6H_5$) auch durch Halogen, $C_1$-$C_6$-Alkoxy, Trifluormethyl, $C_1$-$C_6$-Alkyl, Methylendioxy, Cyan substituiert sein können und D die oben angegebenen Bedeutungen hat;

$R_2$ und $R_3$, die gleich oder verschieden sein können:

Wasserstoff, Halogen, Hydroxy, $C_1$-$C_6$-Alkyl, Trifluormethyl, -CN, $C_1$-$C_6$-Alkoxy, $C_3$-$C_6$-Alkenyloxy, $C_3$-$C_6$-Alkinyloxy, $-NHR_5$, $-NR_5R_6$, $NR_5R_6R_7$ (Bedeutungen $R_5$, $R_6$, $R_7$ wie angegeben), oder die Gruppe -G-Alk-A, wobei Alk und A die angegebenen Bedeutungen haben und G Sauerstoff, Schwefel, NH oder $NR_5$ ist;

$R_4$: Wasserstoff oder Halogen

wobei $R_1$ auch Wasserstoff sein kann, wenn $R_2$ die Gruppe

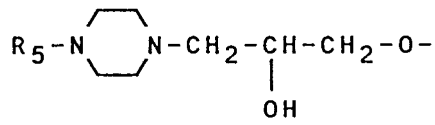

ist und $R_5$ Phenyl, $C_1$-$C_4$-Alkoxy-phenyl oder Diphenylmethyl darstellen und $R_3$ und $R_4$ Wasserstoff sind,
und deren physiologisch verträglichen Säureadditionssalze und quartären Ammoniumsalze, ausgenommen die Verbindungen der Formel I, wo $R_1$ Methyl, Dimethylamino-propyl, Dimethylamino-ethyl, Morpholino-ethyl oder Pyrrolidino-ethyl ist, $R_2$, $R_3$ und $R_4$ Wasserstoff sind und der Phenylring kein Stickstoffatom enthält,
dadurch gekennzeichnet,
daß man in eine Verbindung der Formel

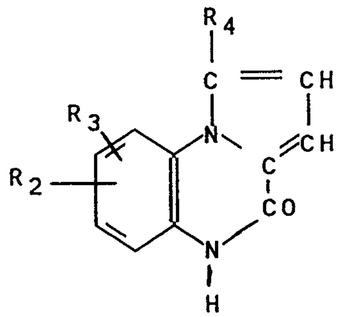

II

worin $R_2$, $R_3$ und $R_4$ die angegebenen Bedeutungen haben können, durch Umsetzung mit einer Verbindung $R_1$Hal, worin $R_1$ die angegebenen Bedeutungen außer der Hydroxygruppe hat, den Rest $R_1$ einführt und/oder in eine Verbindung der Formel II, worin $R_2$ Hydroxy ist, $R_3$ und $R_4$ die angegebenen Bedeutungen haben, durch Umsetzung mit einer Verbindung Hal-Alk-A die Gruppe Alk-A einführt, wobei Alk und A die angegebenen Bedeutungen haben, die erhaltenen Verbindungen gegebenenfalls zwecks Vervollständigung der Reste $R_2$ und $R_3$ und auch $R_1$ alkyliert, acyliert, aminiert und/oder falls $R_1$ eine $C_1$-$C_6$-Alkoxygruppe ist, die Alkylethergruppe zur Hydroxygruppe abspaltet und die erhaltenen Verbindungen gegebenenfalls in ihre Salze überführt.

**3.** Verfahren zur Herstellung von Verbindungen der Formel

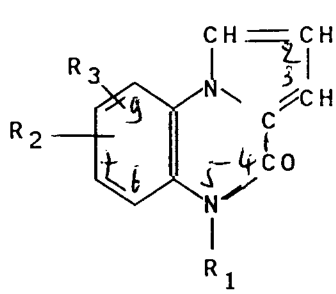

worin der Phenylring entweder in Stellung 9 anstelle einer CH-Gruppe auch ein Stickstoffatom enthalten kann und die Reste $R_1$, $R_2$ und $R_3$ die folgenden Bedeutungen haben:

| | |
|---|---|
| $R_1$: | Wasserstoff |
| $R_2$, $R_3$ (gleich oder verschieden): | Wasserstoff, Halogen, $C_1$-$C_6$-Alkyl, $-CF_3$, -CN, $C_1$-$C_6$-Alkoxy, Amino, $C_2$-$C_6$-Alkanoylamino, $-NO_2$, Hydroxy, $-SO_3H$, $-CO_2H$, -COO-$C_1$-$C_6$-Alkyl, -CONH-$C_1$-$C_6$-Alkyl, -CON($C_1$-$C_6$-Alkyl)2, $-NHR_5$ oder $-NR_5R_6$, wobei $R_5$ und $R_6$ die in Anspruch 1 angegebenen Bedeutungen haben, |

dadurch gekennzeichnet,
daß man eine Verbindung der Formel

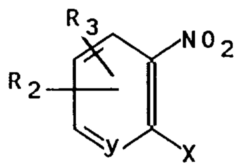

III

worin $R_2$ und $R_3$ die zuvor angegebenen Bedeutungen haben, Y ein Stickstoffatom oder CH bedeutet und X Fluor, Chlor, Brom oder Jod ist mit einer Verbindung der Formel

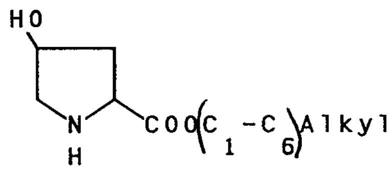

IV

zur Verbindung der Formel

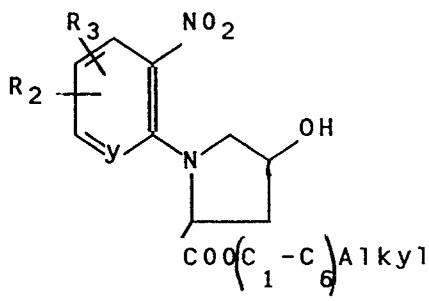

V

umsetzt, diese Verbindung mittels Reduktion zur Verbindung der Formel

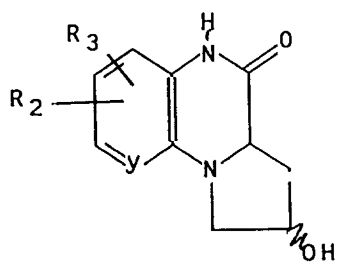

VI

umsetzt und aus der so erhaltenen Verbindung durch Behandlung mit Sauerstoff oder eines sauerstoffliefernden Stoffes die Verbindung der Formel

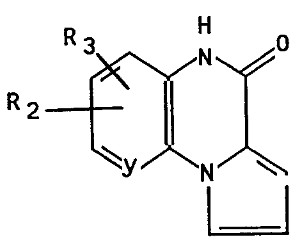

VII

erhält.

4. Verbindungen der Formel gemäß Anspruch 3 erhalten nach Anspruch 3

5. Verfahren nach einem oder mehreren der vorangegangenen Ansprüche, dadurch gekennzeichnet, daß man basische Verbindungen der Formel I in die Salze überführt.

6. Verbindungen der Formel I zur Anwendung als therapeutische Wirkstoffe.

7. Arzneimittel, enthaltend eine Verbindung der allgemeinen Formel I neben üblichen Träger-und/oder Verdünnungs- beziehungsweise Hilfsstoffen.

8. Verfahren zur Herstellung eines Arzneimittels,
dadurch gekennzeichnet,
daß eine Verbindung der allgemeinen Formel 1 mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht wird.

9. Verwendung von Verbindungen der allgemeinen Formel I zur Herstellung von Arzneimitteln.